# EUROPEAN PATENT SPECIFICATION

(11) **EP 3 116 475 B1**
(45) Date of publication and mention of the grant of the patent: **04.11.2020**
(21) Application number: 15731727.2
(22) Date of filing: 12.03.2015
(51) Int. Cl.: A61K 9/00, A61K 31/198, A61K 31/133, A61K 47/18, A61K 47/20, A61K 47/22, A61P 25/16

(54) **DOPA DECARBOXYLASE INHIBITOR COMPOSITIONS**
DOPA-DECARBOXYLASEHEMMERZUSAMMENSETZUNGEN
COMPOSITIONS D'INHIBITEURS DE DOPA-DÉCARBOXYLASE

(30) Priority: 13.03.2014 US 201461952477 P; 09.05.2014 US 201461990967 P
(43) Date of publication of application: 18.01.2017
(60) Divisional application: 20198371.5
(73) Proprietor: Neuroderm Ltd, 7403648 Ness Ziona (IL)
(72) Inventor: YACOBY-ZEEVI, Oron, 6094600 Bitsaron (IL)
(74) Representative: Hoffmann Eitle
(86) International application number: PCT/IL2015/050258
(87) International publication number: WO 2015/136538

(56) References cited:
- WO-A1-2014/141261
- US-A1- 2005 070 608
- US-A1- 2014 051 755
- ERIC J. PAPPERT ET AL: "Clinical/ Scientific Notes - The Stability of Carbidopa in Solution", MOVEMENT DISORDERS, vol. 12, no. 4, 1997, pages 608-623, XP055206267,

## Description

### TECHNICAL FIELD

The present invention provides pharmaceutical compositions of carbidopa and levodopa, containing safe and tolerable concentration of hydrazine.

### BACKGROUND

Parkinson's disease is a degenerative condition characterized by reduced concentration of the neurotransmitter dopamine in the brain. Levodopa (L-dopa or L-3,4-dihydroxyphenylalanine) is an immediate metabolic precursor of dopamine that, unlike dopamine, is able to cross the blood-brain barrier and is most commonly used for restoring the dopamine concentration in the brain. For the past 40 years, levodopa has remained the most effective therapy for the treatment of Parkinson's disease.

However, levodopa has a short half-life in plasma that, even under best common current standard of care, results in pulsatile dopaminergic stimulation. Long-term therapy is therefore complicated by motor fluctuations and dyskinesia that can represent a source of significant disability for some patients. A therapeutic strategy that could ultimately deliver levodopa/dopamine to the brain in a more continuous and physiologic manner would provide the benefits of standard levodopa with reduced motor complications and is much needed by patients suffering from Parkinson's disease and other neurological or movement disorders (Olanow, Mov. Dis., 2008, 23(Suppl. 3), S613-S622). Sustained-release oral levodopa formulations have been developed, but, at best, such preparations have been found to be no more efficacious than standard tablets. Continuous administration of levodopa by intraduodenal administration or infusion has also been attempted by using ambulatory pumps or patches. Such treatments, especially intraduodenal, are extremely invasive and inconvenient.

The metabolic transformation of levodopa to dopamine is catalyzed by the aromatic L-amino acid decarboxylase enzyme, a ubiquitous enzyme with particularly high concentrations in the intestinal mucosa, liver, brain, and brain capillaries. Due to the possibility of extracerebral metabolism of levodopa, it is necessary to administer large doses of levodopa leading to high extracerebral concentrations of dopamine that cause nausea in some patients. Therefore, levodopa is usually administered concurrently with oral administration of a dopa decarboxylase inhibitor, such as carbidopa or benserazide, which reduces by 60-80% the levodopa dose required for a clinical response, and thus prevents certain of its side effects by inhibiting the conversion of levodopa to dopamine outside the brain.

Various oral formulations together with inhibitors of enzymes associated with the metabolic degradation of levodopa are well known, e.g., decarboxylase inhibitors such as carbidopa and benserazide, monoamone oxidase (MAO)-A or MAO-B inhibitors such as moclobemide, rasagiline, selegiline, and safinamide, and catechol-O-methyl transferase (COMT) inhibitors such as tolcapone and entacapone. Currently available oral drugs include SINEMET® and SINEMET®CR sustained-release tablets that include carbidopa or levodopa; MADOPAR® tablets containing levodopa and benserazide; and STALEVO® tablets containing carbidopa, entacapone, and levodopa.

US 2005/070608 A1 discloses liquid compositions of levodopa and carbidopa with low levels of degradants such as hydrazine.

Carbidopa is a non-competitive inhibitor of DOPA decarboxylase. When mixed with levodopa, carbidopa inhibits the peripheral conversion of levodopa to dopamine. This results in increased levodopa available for transport to the CNS. Carbidopa also inhibits the metabolism of levodopa in the GI tract, thus, increasing levodopa bioavailability. It is used in Parkinson's disease to reduce the peripheral effect of dopamine. The loss of the hydrazine functional group represents the major metabolic pathway for carbidopa.

Hydrazine (N₂H₄) and its salts are used in the pharmaceutical industry as an intermediate to produce drugs with different therapeutic effects including decarboxylase inhibitors, antihypertensives, and antibacterials. It has been the cause of severe adverse effects on the central nervous system, liver, and kidneys. In addition to these effects, experimental animals have also shown the following symptoms: loss of body weight, anaemia, hypoglycaemia, fatty degeneration of the liver, and convulsions. Hydrazine has also been shown to cause DNA damage, gene mutations, and chromosome aberrations (Environmental health criteria No. 68 hydrazine (1987)) and has induced tumor growth in mice, hamsters, and rats after oral, intraperitoneal, and inhalation administration (MacEwan, J.D., Vernot, E.H., Haun C.C., et al., Chronic inhalation toxicity of hydrazine: oncogenic effects (1981). Air Force Aerospace Medical Research Laboratory, Wright-Patterson Air Force Base, Ohio, NTIS Springfield VA). Since hydrazine is toxic and thought to be a possible human carcinogen, its presence is limited in some of these drug substances in the monographs of the European Pharmacopoeia (Ph. Eur.).

Accordingly, there is an ongoing and urgent need for liquid formulations and compositions that can achieve continuous dopaminergic stimulation to treat movement disorders such as Parkinson's disease more effectively containing a safe and tolerable amount of hydrazine.

### SUMMARY OF INVENTION

It has now been found that carbidopa formulations containing particular combinations of two antioxidants or o-quinone scavengers, wherein one of those antioxidants is ascorbic acid or a salt thereof, are significantly more stable than those formulations containing just a single antioxidant. As particularly found, particular antioxidant combinations consisting of ascorbic acid or a salt thereof, and one or more additional antioxidants, strongly inhibit carbidopa degradation thus significantly reducing, i.e., limiting, formation of undesired degradation products, in particular 3,4-dihydroxyphenyl-2-methylpropionic acid (herein identified *"**degradant**"*) and hydrazine, and substantially stabilizing the formulations. Surprisingly, it has been further found that such carbidopa formulations can be stored at various temperatures and conditions for long periods of time, more particularly up to several years, wherein a safe and tolerable concentration of hydrazine is maintained.

The invention is defined by the claims. Any subject-matter falling outside the scope of the claims is provided for information purposes only. Further, any references in the description to methods of treatment refer to the compounds, pharmaceutical compositions and medicaments of the present invention for use in a method for treatment of the human or animal body by therapy.

The present invention provides a pharmaceutically acceptable liquid composition comprising: about 0.75% by weight carbidopa; about 6% by weight levodopa; about 12% to about 36% by weight arginine; about 0.5% by weight ascorbic acid or a salt thereof; about 0.1 % to about 0.6% by weight L-cysteine, or a salt thereof, or about 0.01 % to about 1 % by weight N-acetylcysteine (NAC); and optionally, about 0.1% to about 0.5% by weight, preferably about 0.3% by weight, polysorbate 80, wherein said composition, after 1-24 hours at 25°C, comprises less than 0.1 µg/ml hydrazine, as determined by a GCMS method.

The present invention also provides a pharmaceutically acceptable liquid composition comprising: about 0.6% to 1.4% by weight carbidopa; about 6% by weight levodopa; about 15% to about 16% by weight arginine; about 0.5% by weight ascorbic acid or a salt thereof; about 0.4% by weight L-cysteine or about 0.5% by weight N-acetylcysteine (NAC); and about 0.3% by weight polysorbate 80, wherein said composition, after 1-24 hours at 25°C, comprises less than 0.1 µg/ml hydrazine, as determined by a GCMS method.

Further, the present invention also provides a pharmaceutically acceptable liquid composition according to any one of claims 1 to 8, for use in treating a disease, disorder or condition associated with loss of dopamine or dopaminergic neurons.

In one aspect, it is provided a pharmaceutical composition comprising carbidopa, at least two antioxidants, and a pharmaceutically acceptable carrier, wherein one of said antioxidants is ascorbic acid or a salt thereof, and said composition comprises less than 1 µg/ml hydrazine, as determined by a gas chromatography-mass spectrometry (GCMS), or less than 5% by weight 3,4-dihydroxyphenyl-2-methylpropionic acid relative to the initial amount of carbidopa, as determined by high-performance liquid chromatography (HPLC). Particular such pharmaceutical compositions comprise 0.1% to 10%, preferably 0.5% to 6%, by weight carbidopa, and/or 0.1% to 10%, preferably 0.2% to 2%, by weight, ascorbic acid or a salt thereof.

In a particular such aspect, the pharmaceutical composition further comprises (i) levodopa; (ii) arginine, meglumine, or a combination thereof; or (iii) both (i) and (ii); and may optionally further comprise a surfactant. Particular such pharmaceutical compositions comprise either less than 1% or 1% to 20%, preferably 2% to 16%, by weight, levodopa; and/or 0.1% to 42%, preferably 2% to 40%, by weight, arginine, meglumine, or a combination of both arginine and meglumine.

Particular pharmaceutical compositions comprise carbidopa; ascorbic acid or a salt thereof; at least one additional antioxidant other than said ascorbic acid or salt thereof, e.g., L-cysteine or a salt thereof, N-acetylcysteine (NAC) or a salt thereof, glutathione or a salt thereof, diacetylcystine or a salt thereof, or sodium bisulfite; levodopa; arginine, meglumine, or a combination thereof; and optionally a surfactant, e.g., polysorbate 80.

More particular such compositions comprise (i) 0.1% to 10%, preferably 0.5% to 6%, by weight, carbidopa; (ii) 0.1% to 10%, preferably 0.2% to 2%, by weight, ascorbic acid or a salt thereof; (iii) 0.001% to 5%, by weight, L-cysteine or salt thereof; 0.001% to 5%, by weight, NAC; or 0.01% to 2%, by weight, sodium bisulfite; (iv) either less than 1% or 1% to 20%, preferably 2% to 16%, by weight, levodopa; (v) 0.1% to 42%, preferably 2% to 40%, by weight, arginine, meglumine, or a combination thereof; and optionally (vi) 0.01% to 5%, by weight, polysorbate 80, wherein the composition comprises less than 1 µg/ml, preferably less than 0.5 µg/ml, more preferably less than 0.1 µg/ml, hydrazine.

As shown herein, the pharmaceutical compositions are highly stable, wherein the particular combination of antioxidants stabilizes the carbidopa thereby minimizing the degradation of the carbidopa thus inhibiting formation of degradant and hydrazine. Consequently, those compositions can be stored at various conditions, e.g., at a temperature ranging from -20°C to 25°C, without being substantially degraded, wherein the hydrazine content of the compositions is maintained at less than 1 µg/ml, preferably less than 0.1 µg/ml, after 1-24 hours, 1-30 days, 1-12 months, or 1-3 years.

In another aspect, the present disclosure relates to a method for treating a disease, disorder or condition associated with loss of dopamine or dopaminergic neurons, e.g., Parkinson's disease, in a patient, more specifically a mammal in general or a human in particular, said method comprising administering to said patient an effective amount of a pharmaceutical composition as defined above. The method may include substantially continuous administration of the composition.

In another aspect, the present invention relates to a pharmaceutically acceptable liquid composition as defined in any one of claims 1 to 8, for use in treatment of a disease, disorder or condition associated with loss of dopamine or dopaminergic neurons, e.g., Parkinson's disease.

In yet another aspect, the present invention relates to use of a pharmaceutical composition as defined above, for the preparation of a medicament for treatment of a disease, disorder or condition associated with loss of dopamine or dopaminergic neurons, e.g., Parkinson's disease.

In a further aspect, the present disclosure provides a kit comprising at least one, i.e., 1, 2, 3 or more, containers each containing a pharmaceutical composition as defined above, wherein said composition is present in an amount sufficient to treat a patient, i.e., a mammal or a human, for a disease, disorder or condition associated with loss of dopamine or dopaminergic neurons, e.g., Parkinson's disease, for at least 1, 2, 3, 4, or 5 days; 1, 2, 3, or 4 week; 1 to 12 (e.g., 1, 2, 3, 6, 9, or 12) months; or 1 to 20 (e.g., 1, 2, 3, 4, 5, 6, 8, 10, or 12) years. In certain embodiments, the composition is present in separate dosages.

In certain embodiments of any of these aspects, the pharmaceutical composition, method, or kit further comprises, or comprises the use of, a second active agent. Such a second agent may be a catechol-O-methyl transferase (COMT) inhibitor, e.g., tolcapone, entacapone, or a pharmaceutically acceptable salt thereof.

### BRIEF DESCRIPTION OF THE FIGURES

**Fig. 1** shows a graph depicting the main impurity ("degradant") at retention time of about 14.5±0.2 min.
**Figs. 2A-2B** show graphs depicting typical MS spectrum in negative (**2A**) and positive (**2B**) mode of collected main impurity peak from a formulation sample.
**Figs. 3A-3B** show graphs depicting typical MS/MS daughter scan (ion M/Z=179) spectrum (**3A**) and parent (ion M/Z=105) spectrum (**3B**) in collected main impurity peak from a formulation sample.

### DETAILED DESCRIPTION OF THE INVENTION

The present invention provides pharmaceutical compositions of carbidopa that are highly stable for a long period of time, more particularly for days, weeks, months or years, and contain very low content, i.e., a safe and tolerable amount, of hydrazine. While carbidopa-based compositions containing an antioxidant have already been disclosed in the prior art, the pharmaceutical compositions of the present invention are stabilized by a particular combination of two antioxidants, wherein one of those antioxidants is ascorbic acid or a salt thereof, and the other antioxidants of the antioxidant combination each independently is selected from L-cysteine or NAC. Such compositions comprise active agents other than carbidopa, in particular levodopa, as well as further ingredients for further stabilizing the composition, i.e arginine (Arg; L-Arg), and may comprise one or more surfactants.

The pharmaceutical composition of the present invention comprises less than or 0.025 µg/ml hydrazine, as determined by a GCMS method, particularly less than 0.05 µg/ml hydrazine, as determined by a GCMS method ; or less than 5%, 4%, 3%, 2%, 1%, 0.5%, 0.3%, 0.2%, 0.1%, or 0.05%, by weight, degradant relative to the initial amount of carbidopa, as determined by HPLC.

In certain aspects, a pharmaceutical composition comprises 0.1% to 10% by weight carbidopa. In particular such aspects, the pharmaceutical composition comprises 0.5% to 6%, preferably 0.75% to 4%, more preferably 0.75%, 1.4%, 3%, 3.3% or 4%, by weight, carbidopa.

The pharmaceutical composition of the present invention comprises a combination of two antioxidants according to the claims, wherein one of those antioxidants is ascorbic acid or a salt thereof. Examples of ascorbic acid salts include, without being limited to, sodium ascorbate, potassium ascorbate and calcium ascorbate, wherein sodium ascorbate is preferred.

The pharmaceutical composition of the present invention comprises 0.5 % , by weight, ascorbic acid or a salt thereof such as sodium ascorbate, potassium ascorbate or calcium ascorbate.

According to the present invention, each one of the antioxidants comprised within the pharmaceutical composition of the present invention, other than ascorbic acid or salt thereof, is an antioxidant that, together with ascorbic acid or a salt thereof, provides a combination that can strongly inhibit degradation of carbidopa thus minimize hydrazine formation, and consequently substantially stabilize said composition for a sufficient period of time, e.g., for hours, days, weeks, months or years.

Each one of the antioxidants other than ascorbic acid or salt thereof independently is selected from L-cysteine (L-Cys) or a salt thereof such as cysteine hydrochloride, or NAC. In particular such embodiments, the pharmaceutical composition of the present invention comprises (i) 0.1% to 0.6%, 0.3%, or 0.4%, by weight, L-cysteine or a salt thereof such as cysteine hydrochloride; or 0.01% to 1%, more preferably 0.1%, 0.2%, 0.3%, or 0.4%, by weight, NAC.

In certain aspects, a pharmaceutical composition comprises 0.1% to 10%, by weight, carbidopa; 0.1% to 10%, by weight, ascorbic acid or a salt thereof such as sodium ascorbate, potassium ascorbate or calcium ascorbate; and (i) 0.001% to 5%, by weight, L-cysteine or a salt thereof such as cysteine hydrochloride; or (ii) 0.001% to 5%, by weight, NAC; or (iii) 0.001% to 5%, by weight, glutathione; or (iv) 0.001% to 5%, by weight, diacetylcystine or a salt thereof; or (v) 0.01% to 2%, by weight, sodium bisulfite. Particular such aspects are those wherein the composition comprises 0.5% to 6%, preferably 0.75% to 4%, more preferably 0.75%, 1.4%, 3%, 3.3% or 4%, by weight, carbidopa; 0.2% to 2%, preferably 0.4% to 1.3%, more preferably 0.5%, 0.6%, 0.75%, 0.85%, 1.0%, or 1.2%, by weight, ascorbic acid or a salt thereof; and (i) 0.01% to 1%, preferably 0.1% to 0.6%, by weight, L-cysteine or a salt thereof; (ii) 0.01% to 1%, preferably 0.1% to 0.4%, by weight, NAC; or (iii) 0.075% to 0.75%, by weight, sodium bisulfite.

In a particular such aspect, a pharmaceutical composition further comprises (i) levodopa (and/or an ester thereof); (ii) arginine, meglumine, a salt of the aforesaid, or a combination thereof; or (iii) both (i) and (ii); and optionally further comprises a surfactant. Particular such compositions do not comprise levodopa.

In certain aspects, a pharmaceutical composition comprises carbidopa and at least two antioxidants, each as defined in any one of the aspects above, and further comprises levodopa. Certain particular such compositions comprise less than 1% (e.g., less than 0.5%, 0.25%, 0.1%, 0.05%, or 0.01%), by weight, levodopa, while other compositions comprise 1% to 20%, preferably 2% to 16% (e.g., 2% to 8%, 4% to 8%, 5% to 7%, 8% to 16%, 10% to 15%, 12% to 15%), more preferably 4%, 6%, 12% or 13.2%, by weight, levodopa.

In certain aspects, a pharmaceutical composition comprises carbidopa and at least two antioxidants, each as defined in any one of the aspects above, and further comprises arginine, meglumine, or a combination thereof. Particular such compositions comprise 0.1% to 42% (e.g., 0.1% to 40%, 10% to 25% 13% to 18%, 14% to 16%, 12% to 40%, 25% to 40%, 30% to 38%, 10% to 20%, and 20% to 42%), preferably 2% to 40% or 10% to 38%, more preferably 12% to 36% or 15.2% to 32%, by weight, arginine, meglumine, or a combination thereof. It should be understood that while certain particular such compositions comprise arginine only, other particular compositions comprise meglumine only, and further particular compositions comprise both arginine and meglumine.

In certain aspects, a pharmaceutical composition comprises carbidopa; at least two antioxidants; and (i) levodopa; (ii) arginine, meglumine, or a combination thereof; or (iii) both (i) and (ii), each as defined in any one of the aspects above, and further comprises a surfactant, e.g., a nonionic surfactant such as polysorbate 20 [polyoxyethylene (20) sorbitan monolaurate], polysorbate 40 [polyoxyethylene (20) sorbitan monopalmitate], polysorbate 60 [polyoxyethylene (20) sorbitan monostearate], polysorbate 80 [polyoxyethylene (20) sorbitan monooleate; Tween® 80], or a combination thereof. Particular such compositions comprise 0.01% to 5%, preferably 0.1% to 0.5% or 0.2% to 0.4%, more preferably 0.3%, by weight, of a nonionic surfactant as listed above, preferably polysorbate 80.

In certain aspects, a pharmaceutical composition comprises carbidopa and at least two antioxidants, each as defined in any one of the aspects above, and further comprises 1% to 20%, by weight, levodopa; and 0.1% to 42%, by weight, arginine, meglumine, or a combination thereof. Particular such aspects are those wherein the composition comprises 2% to 16% (e.g., 2% to 8%, 4% to 8%, 5% to 7%, 8% to 16%, 10% to 15%, 12% to 15%), preferably 4%, 6%, 12% or 13.2%, by weight, levodopa; and 0.1% to 42% (e.g., 0.1% to 40%, 10% to 25% 13% to 18%, 14% to 16%, 12% to 40%, 25% to 40%, 30% to 38%, 10% to 20%, and 20% to 42%), preferably 10% to 38%, more preferably 12% to 36% or 15.2% to 32%, by weight, arginine, meglumine, or a combination thereof. In more particular such aspects, the composition further comprises a surfactant, e.g., polysorbate 80, more specifically 0.1% to 0.5% or 0.2% to 0.4%, preferably 0.3%, by weight, polysorbate 80.

In certain aspects, a pharmaceutical composition comprises (i) 0.1% to 6% by weight carbidopa; (ii) 0.1% to 10% by weight ascorbic acid or a salt thereof; (iii) 0.01% to 1% by weight L-cysteine or a salt thereof, NAC, or glutathione; (iv) 0% to 16% by weight levodopa; and (v) 0.1% to 40% by weight arginine.

In certain aspects, a pharmaceutical composition thus comprises (i) 0.1% to 10%, by weight, carbidopa; (ii) 0.1% to 10%, by weight, ascorbic acid or a salt thereof such as sodium ascorbate, potassium ascorbate or calcium ascorbate; (iii) 0.001% to 5%, by weight, L-cysteine or a salt thereof such as cysteine hydrochloride; or 0.001% to 5%, by weight, NAC; or 0.001% to 5%, by weight, glutathione; or 0.001% to 5%, by weight, diacetylcystine or a salt thereof; or 0.01% to 2%, by weight, sodium bisulfite; (iv) 1% to 20%, by weight, levodopa; (v) 0.1% to 42%, by weight, arginine, meglumine, or a combination thereof; and optionally (vi) 0.01% to 5%, by weight, polysorbate 80, wherein the composition comprises less than 1 µg/ml, preferably less than 0.5 µg/ml, more preferably less than 0.1 µg/ml, hydrazine. In particular such aspects , the composition comprises (i) 0.5% to 6%, preferably 0.75% to 4%, more preferably 0.75%, 1.4%, 3%, 3.3%, or 4%, by weight, carbidopa; (ii) 0.2% to 2%, preferably 0.4% to 1.3%, more preferably 0.5%, 0.6%, 0.75%, 0.85%, 1.0%, 1.2%, or 1.3%, by weight, ascorbic acid or a salt thereof; (iii) 0.01% to 1%, preferably 0.1% to 0.6%, by weight, L-cysteine or a salt thereof; 0.01% to 1%, preferably 0.1% to 0.4%, by weight, NAC; or 0.075% to 0.75%, by weight, sodium bisulfite; (iv) 2% to 16%, preferably 4% to 14%, more preferably 4%, 6%, 12% or 13.2%, by weight, levodopa; (v) 2% to 42%, preferably 10% to 38%, more preferably 12% to 36% or 15.2% to 32%, by weight, arginine, meglumine, or a combination thereof; and optionally (vi) 0.1% to 0.5% or 0.2% to 0.4%, preferably 0.3%, by weight, polysorbate 80. In other particular such aspects, the composition comprises (i) 0.1% to 3%, preferably 0.5% to 2% or 0.6% to 1.5%, by weight, carbidopa; (ii) 0.1% to 10%, preferably 0.1% to 2% or 0.3% to 0.7%, by weight, ascorbic acid or a salt thereof; (iii) 0.001% to 5%, preferably 0.1% to 2% or 0.3% to 0.5%, by weight, L-cysteine or a salt thereof, NAC, glutathione, or diacetylcystine; (iv) 2% to 8%, preferably 4% to 8% or 5% to 7%, by weight, levodopa; (v) 10% to 25%, preferably 13% to 18% or 14% to 16%, by weight, arginine, meglumine, or a combination thereof; and optionally (vi) 0.01% to 5% or 0.1% to 0.5%, or 0.3%, by weight, polysorbate 80. In further particular such aspects , the composition comprises (i) 1% to 4%, preferably 1.2% to 4% or 2% to 4%, by weight, carbidopa; (ii) 0.1% to 10%, preferably 0.1% to 2% or 0.3% to 0.7%, by weight, ascorbic acid or a salt thereof; (iii) 0.001% to 1%, preferably 0.1% to 1% or 0.2% to 0.5%, by weight, L-cysteine or a salt thereof, NAC, glutathione, or diacetylcystine; (iv) 8% to 16%, preferably 10% to 15% or 12% to 15%, by weight, levodopa; (v) 12% to 40%, preferably 25% to 40% or 30% to 38%, by weight, arginine, meglumine, or a combination thereof; and optionally (vi) 0.01% to 5% or 0.1% to 0.5%, or 0.3%, by weight, polysorbate 80. In still a further particular such aspect, the composition comprises (i) 0.1% to 1.5% by weight carbidopa; (ii) 0.1% to 1.5%, preferably 0.4% to 0.6% or 0.4% to 1%, by weight, ascorbic acid or a salt thereof; (iii) 0.1% to 0.7% by weight L-cysteine or a salt thereof, or NAC; (iv) 4% to 8% by weight levodopa; (v) 10% to 20% by weight arginine; and optionally (vi) 0.1% to 0.5% by weight polysorbate 80. In yet a further particular such aspect, the composition comprises (i) 1% to 4% by weight carbidopa; (ii) 0.1% to 1.5%, preferably 1% to 1.4%, by weight, ascorbic acid or a salt thereof; (iii) 0.1% to 1%, preferably 0.1 to 0.5%, by weight, L-cysteine or a salt thereof, or NAC; (iv) 8% to 16% by weight levodopa; (v) 20% to 40% by weight arginine, meglumine, or a combination thereof.

In more particular aspects, a pharmaceutical composition comprises (i) 12% to 15% by weight levodopa, 1.2% to 4% by weight carbidopa, 32% to 42%, e.g., 32%, 33%, 34%, 35% or 36%, by weight arginine or meglumine, 1% to 1.3% by weight sodium ascorbate, 0.1-0.5% by weight L-cysteine (or cysteine hydrochloride) or NAC, and optionally polysorbate 80, e.g., 0.3% by weight; or (ii) 6% by weight levodopa, 0.6% to 1.4% by weight carbidopa, 15% to 16% by weight arginine, 0.5% by weight ascorbic acid, 0.3% by weight polysorbate 80, and 0.5% by weight NAC or 0.4% by weight L-cysteine.

**Table 1: Specific pharmaceutical compositions exemplified herein (compositions 6-9 are according to the invention)**

| **No.** | **CD** | **LD** | **L-Arg** | **Meglumine** | **Ascorbic acid Na-ascorbate** | **L-Cys** | **NAC** | **Tween®80** |
|---|---|---|---|---|---|---|---|---|
| **1** | 3% | 12% | 32% | | 1.2% Na-ascorbate | 0.3%¹ | | |
| **2** | 3.3% | 13.2% | 36% | | 1.3% Na-ascorbate | 0.3%¹ | | |
| **3** | 3.3% | 13.2% | | 36% | 1.3% Na-ascorbate | 0.3%¹ | | |
| **4** | 3% | 12% | | 32% | 1.2% Na-ascorbate | | 0.3% | |
| **5** | 3% | 12% | 32% | | 1.2% Na-ascorbate | | 0.3% | |
| **6** | 1.4% | 6% | 15.5% | | 0.5% ascorbic acid | 0.4% | | 0.3% |
| **7** | 1.4% | 6% | 15.5% | | 0.5% ascorbic acid | | 0.5% | 0.3% |
| **8** | 0.75% | 6% | 15.2% | | 0.5% ascorbic acid | 0.4% | | 0.3% |
| **9** | 0.75% | 6% | 15.2% | | 0.5% ascorbic acid | | 0.5% | 0.3% |

| | | | | | | | | |
|---|---|---|---|---|---|---|---|---|
| ¹ Or cysteine hydrochloride. | | | | | | | | |

In certain specific aspects, a pharmaceutical composition is one of those exemplified herein and listed in **Table 1.** These compositions comprise (i) 12% by weight levodopa, 3% by weight carbidopa, 32% by weight arginine, 1.2% by weight sodium ascorbate, and 0.3% by weight L-cysteine or cysteine hydrochloride; (ii) 13.2% by weight levodopa, 3.3% by weight carbidopa, 36% by weight arginine, 1.3% by weight sodium ascorbate, and 0.3% by weight L-cysteine or cysteine hydrochloride; (iii) 13.2% by weight levodopa, 3.3% by weight carbidopa, 36% by weight meglumine, 1.3% by weight sodium ascorbate, and 0.3% by weight L-cysteine or cysteine hydrochloride; (iv) 12% by weight levodopa, 3% by weight carbidopa, 32% by weight meglumine, 1.2% by weight sodium ascorbate, and 0.3% by weight NAC; (v) 12% by weight levodopa, 3% by weight carbidopa, 32% by weight arginine, 1.2% by weight sodium ascorbate, and 0.3% by weight NAC; (vi) 6% by weight levodopa, 1.4% by weigh carbidopa, 15.5% by weight arginine, 0.5% by weight ascorbic acid, 0.4% by weight L-cysteine and 0.3% by weight polysorbate 80; (vii) 6% by weight levodopa, 1.4% by weigh carbidopa, 15.5% by weight arginine, 0.5% by weight ascorbic acid, 0.5% by weight NAC and 0.3% by weight polysorbate 80; (viii) 6% by weight levodopa, 0.75% by weigh carbidopa, 15.2% by weight arginine, 0.5% by weight ascorbic acid, 0.4% by weight L-cysteine and 0.3% by weight polysorbate 80; or (ix) 6% by weight levodopa, 0.75% by weigh carbidopa, 15.2% by weight arginine, 0.5% by weight ascorbic acid, 0.5% by weight NAC and 0.3% by weight polysorbate 80. The compositions of (i)-(v) above may further comprise polysorbate 80, e.g., 0.3% by weight.

Pharmaceutical compositions according to the present invention may include further antioxidants such as di-tert-butyl methyl phenols, tert-butyl-methoxyphenols, polyphenols, tocopherols, and ubiquinones, e.g., caffeic acid; and/or a glucose amine. Compositions according to the invention may also include a tyrosinase inhibitor such as, without being limited to, captopril, methimazole, quercetin, arbutin, aloesin, N-acetylglucoseamine, retinoic acid, α-tocopheryl ferulate, Mg ascorbyl phosphate (MAP), substrate analogues, e.g., sodium benzoate, and L-phenylalanine, Cu⁺⁺ chelators, e.g., Na₂-EDTA, Na₂-EDTA-Ca, DMSA (succimer), DPA (D-penicillamine), trientine-HCl, dimercaprol, clioquinol, sodium thiosulfate, TETA, TEPA, curcumin, neocuproine, tannin, and cuprizone. The compositions may further include pharmaceutically acceptable fillers, carriers, diluents or adjuvants, as well as other inert ingredients and excipients.

In certain aspects, a pharmaceutical composition comprises carbidopa; at least two antioxidants; levodopa; arginine, meglumine, or a combination thereof; and optionally a surfactant, each as defined in any one of the aspects above, wherein the composition has a pH of 9.1 to 10, preferably 9.4-9.8, more preferably 9.6 to 9.8.

As stated above, the pharmaceutical compositions of the present invention are highly stable due to the particular antioxidant combination which stabilizes the carbidopa in the composition and strongly inhibits its degradation to degradant and hydrazine. Moreover, these compositions can be stored at various conditions and temperatures, e.g., at a temperature of up to 25°C, for long periods of time, more particularly up to several years, while maintaining a safe and tolerable concentration of hydrazine.

The pharmaceutical composition of the present invention according to any one of the embodiments defined above comprises less than 0.1 µg/ml, hydrazine, as determined by a GCMS method. It may comprise less than 5%, preferably less than 1%, more preferably less than 0.75%, 0.6%, 0.5%, 0.4%, 0.3%, 0.25%, 0.2%, 0.1%, 0.05%, or 0.01%, by weight degradant relative to the initial amount of carbidopa, as determined by HPLC, after storage for 1, 2, 3, 4, 5, 6, 8, 10, 12, 14, 16, 18, 20, 22, or 24 hours; 1, 2, 3, 4, 5, 6, 7, 8, 9, 10, 11, 12, 13, 14, 21, 28, or 30 days; 1, 2, 3, 4, 5, 6, 7, 8, 9, 10, 11, or 12 months; or 1, 1.5, 2, 2.5, or 3 years, at a temperature in a range of -20°C to 25°C, e.g., at -20°C, 2-8°C, or 25°C.

The pharmaceutical compositions provided by the present invention may be prepared by conventional techniques, e.g., as described in Remington: The Science and Practice of Pharmacy, 19th Ed., 1995, following one of the procedures described in the Experimental section herein. For example, such compositions may be prepared by mixing all the ingredients, i.e., carbidopa, antioxidants, and levodopa, arginine and surfactant, all in the form of powders, in amounts as disclosed above, to form a powder mixture. Water can then be added to the mixture to form a suspension. The water can be pre-heated or the suspension formed can be heated at a temperature and for a time sufficient to dissolve the mixture, e.g., to 40°C to 100°C, 40°C to 80°C, or 60°C to 90°C, e.g., 65±5°C, 72±5°C or 73±3°C, e.g., by adding pre-heated water and/or by placing the mixture in a hot water bath for, e.g., up to 3, 5, 10, 20, 30, 40, 50, 60 minutes, or more, to form a solution, with optional stirring. This is followed by cooling the solution to form the composition. N₂ can be provided the head space of the container. Specific methods of preparation are described in Example 1 below. The pharmaceutical compositions disclosed herein can be sterilized, e.g., using 0.2 µm filters such as nylon-based filters or polyvinylidene difluoride (PVDF) membranes.

The pharmaceutical composition of the invention is formulated as a liquid. Such composition may be formulated for any suitable route of administration, but it is preferably formulated for subcutaneous, transdermal, intradermal, intravenous, intramuscular, intratracheal, intrathecal, intraduodenal, or oral, administration. The compositions may also be formulated for inhalation, or for direct absorption through mucous membrane tissues.

In another aspect, the present invention relates to a method for treatment of a disease, disorder or condition associated with loss of dopamine or dopaminergic neurons in an individual in need thereof, said method comprising administering to said individual an effective amount of a pharmaceutical composition as defined in any one of the embodiments above, i.e., a carbidopa-based composition containing safe and tolerable concentration of hydrazine. The disease, disorder or condition associated with loss of dopamine or dopaminergic neurons may be a neurological or movement disorders including restless leg syndrome, Parkinson's disease, secondary Parkinsonism, Huntington's disease, Shy-Drager syndrome, and conditions resulting from brain injury including carbon monoxide or manganese intoxication. In one particular embodiment, the neurological disorder is Parkinson's disease.

According to the method of the present invention, the pharmaceutical composition can be administered over a defined time period, e.g., days, weeks, months, or years; and can be effected by any appropriate route, e.g., subcutaneously, transdermally, intravenously, intramuscularly, intradermally, intratracheally, intrathecally, intraduodenally, or orally, as well as by inhalation, or direct absorption through mucous membrane tissues.

In certain embodiments, the administration of the pharmaceutical composition according to the method of the present invention is substantially continuous, e.g., subcutaneously or transdermally. The term "substantially continuous", as used herein, means that a single dose of the composition is being administered to said patient or individual over a particular predetermined period of time, e.g., for a period of at least 10, 20 or 30 minutes, 1 hour, 2 hours, 4, hours, 6 hours, 8 hours, 12 hours, 15 hours, 18 hours, 21 hours, or 24 hours, rather than as a bolus, e.g., as a pill taken orally or a bolus injection. Substantially continuous administration of these pharmaceutical compositions can be achieved using, e.g., a transdermal patch or a pump device that continuously administers the composition to the patient over time.

In certain embodiments, liquid compositions according to the invention may be administered at a rate of 0.01 ml/hour/site to 0.4 ml/hour/site, e.g., 0.16 ml/hour/site to 0.24 ml/hour/site. Such rates may be constant throughout the day and night or varied according to patient's need, e.g., may reflect a patient resting or sleeping schedule and waking or higher activity level schedule. Such pharmaceutical compositions may thus be administered, e.g., at a rate of 0.32 ml/hour/site in the morning (e.g., for 2-4 hours before waking), 0.24 ml/hour/site during the daytime or activity time (e.g., for 10 to 12 hours), and/or 0.08 ml/hour/site at rest or at night. In other embodiments, such compositions be administered, e.g., intraduodenally, at a rate of 1.0 ml/hour during the daytime or activity time (e.g., for 2-3 hours before waking and for 10 to 12 hours thereafter), and 0 to 0.5 ml/hour at rest or at night. In further embodiments, such compositions may be administered at a rate of 1.25 ml/hour during the daytime or activity time (e.g., for 2-3 hours before or after waking and for 10 to 14 hours thereafter), and 0 to 0.5 ml/hour (e.g., 0.5±0.25 ml/hour) at rest or night. In still further embodiments, such compositions may be administered at a rate of 0.1 to 1000 µl/hour/site; or at a volume of 2 to 10 ml/24hour/site, preferably 4 to 6 ml/24hour/site; or at a dose of 80 to 800 mg levodopa/day and 20 to 200 mg carbidopa/day; or at a rate of 240 to 360 mg levodopa and 60 to 90 mg carbidopa/day/site.

In certain embodiments, a pharmaceutical composition according to the invention may be substantially continuously administered, e.g., using a pump for subcutaneous infusion (e.g., insulin pump) at an average rate of 10-1000 µl/hour (e.g., 10-250 µl/hour), 300±100 µl/hour, or 200±40 µl/hour continuously for 24 hours; 440±200 µl/hour or 200±50 µl/hour continuously for 16 hours (during waking hours) and 0 to 80 µl/hour or 0 to 200 µl/hour for 8 hours (at night); or using a transdermal patch. Substantially continuously administering the composition to a patient can be doubled or tripled by using more than one pump, patch, or infusion site. In certain embodiments, substantially continuously administering using, e.g., a liquid composition, can be at an average rate of 0.2-2 µl/hour, or 1±0.5 µl/hour continuously for 24 hours; 1±0.5 µl/hour continuously for 16 hours (during waking hours) and 0 to 0.5 µl/hour for 8 hours (at night), via a pump, transdermal patch, or a combination of delivery devices that are suitable for, e.g., subcutaneous, intravenous, intrathecal, and/or intraduodenal administration.

In certain embodiments, the pharmaceutical composition of the invention is used for treatment of said neurological disorder or a movement disorder by acute and immediate administration, e.g., by inhalation or injection.

The term "pharmaceutically acceptable carrier" or "pharmaceutically acceptable excipient" as used herein interchangeably refers to any and all solvents, dispersion media, preservatives, antioxidants, coatings, isotonic and absorption delaying agents, and the like, that are compatible with pharmaceutical administration. The use of such media and ingredients for pharmaceutically active substances is well-known in the art. It should be understood that the compositions of the invention can also contain other active agents providing supplemental, additional, or enhanced therapeutic functions.

The term "acceptable" with respect to a carrier or an excipient comprised within a pharmaceutical composition refers to any carrier, ingredient or molecular entity that do not produce an adverse, allergic or other untoward reaction when administered to a mammal or human as appropriate. For human administration, compositions should meet sterility, pyrogenicity, and general safety and purity standards as required by, e.g., the U.S. Food and Drug Administration (FDA) or the European Medicines Agency (EMA).

The term "physiologically acceptable pH" is understood to mean a pH of, e.g., a formulation or composition that facilitates administration of the formulation or composition to a patient without significant adverse effects, e.g., a pH of 4 to 9.8 (for example, 4±0.3 to 9.5±0.3).

The term "ambient temperature" as used herein refers to a temperature ranging from 10°C to 30°C. In particular embodiments, ambient temperature can be 25°C.

Percentages disclosed herein with respect to the pharmaceutical compositions of the invention are by weight unless indicated otherwise.

In still another aspect, the present invention relates to a pharmaceutical composition as defined above, i.e., a carbidopa-based composition containing safe and tolerable concentration of hydrazine, for use in treatment of a disease, disorder or condition associated with loss of dopamine or dopaminergic neurons, e.g., Parkinson's disease.

In yet another aspect, the present invention relates to use of a pharmaceutical composition as defined above, i.e., a carbidopa-based composition containing safe and tolerable concentration of hydrazine, for the preparation of a medicament for treatment of a disease, disorder or condition associated with loss of dopamine or dopaminergic neurons, e.g., Parkinson's disease.

In a further aspect, the present disclosure provides a kit comprising at least one, i.e., 1, 2, 3 or more, containers each containing a pharmaceutical composition according to any one of the embodiments above, wherein said composition is present in an amount sufficient to treat an individual in need thereof for a disease, disorder or condition associated with loss of dopamine or dopaminergic neurons, e.g., Parkinson's disease, for at least 1 day, 1 week, 1 month, 2 months, 6 months, or 1 year. The containers comprised within the kit of the invention may be, e.g., pre-filled cartridges or vials suitable for use by a patient or physician.

In certain embodiments, the kit thus comprises a pre-filled cartridge containing a pharmaceutical composition as defined above, e.g., a pre-filled cartridge containing a single dose or a dose suitable for a single administration or multiple administrations to a patient of said composition, and optionally instructions for use. Such containers, vials, pre-filled syringes, or the like, may include, e.g., 1-10 ml of a disclosed composition. In a particular embodiment, the kit comprises one or more pre-filled vials, containers or syringes, each containing a disclosed liquid pharmaceutical composition in an amount suitable for filling a syringe pump or patch pump, e.g., 1-10 ml, 1-2 ml, 2-5 ml, 1-2 ml, or 4-10 ml of a disclosed composition.

Considering the increased stability of the compositions of the present invention, particular kits comprise a supply of a composition in an amount sufficient for at least 1, 2, 3, 4, or 5 days; 1, 2, 3, or 4 weeks; 1, 2, 3, 4, 6, or 9 months; or 1 or 1.5 years, of administration to a patient, which can be packaged, e.g., into suitable dosage (e.g., unit dosage) compositions. Such kits can optionally include instructions for their use. For example, a kit for daily use may include one, two or more containers or vials of a disclosed composition, an infusion set, and a disposable patient delivery unit, e.g., syringe.

The invention now being generally described, will be more readily understood by reference to the following examples which are included merely for purposes of illustration of certain aspects and embodiments of the present invention, and are not intended to limit the invention in any way.

### EXAMPLES

### Example 1. Formulation Preparation Procedure

Levodopa (LD) and carbidopa (CD) formulations can be prepared as follows:
***Method #1 (L-Arg** solution)*: L-Arg and Na-bisulfite (Na-Bis) were dissolved in water. The solution was added to the LD and CD powders. The mixture was heated with stirring for 13 min at 75°C until fully dissolved. The LD/CD solution was kept at room temperature (RT) for 10 min to cool down.
*Method #2 (all powders together):* All powders (LD, CD, and L-Arg) were weighed, and water with Na-Bis was added. The suspension was heated with stirring for 13 min at 75°C until fully dissolved. The LD/CD solution was kept at RT for 10 min to cool down.
***Method #3 (same as #2 without Na-Bis pre-heating)***: All powders (LD, CD, and L-Arg) were weighed together and water was added. The suspension was heated with stirring for 13 min at 75°C until fully dissolved. The LD/CD solution was kept at RT for 10 min to cool down.
***Method #4 (preparation in steps)*:** LD and the respective amount of L-Arg were weighed; water and Na-Bis solution were added. The suspension was heated for 7 min at 75°C until fully dissolved, followed by 7 min at RT. CD and the respective amount of L-Arg were weighed and added to the LD/Arg solution at 60°C until fully dissolved. Finally, extra L-Arg was added.
***Method #5 (same as #4 without Na-Bis pre-heating)***: LD and the respective amount of L-Arg were weighed; water was added. The suspension was heated for 7 min at 75°C until fully dissolved followed by 7 min at RT. CD and the respective amount of L-Arg were weighed and added to the LD/Arg solution at 60°C until fully dissolved. Finally, extra L-Arg was added.

After cooling down, all formulations from all methods were divided in to 3 vials, and water, Na-Bis solution, or Na-Bis-Arg solution was added to each vial.

### Example 2. Identification of the main degradant in CD containing formulations

Liquid formulations with levodopa, carbidopa, and arginine were prepared using the procedure outlined in Example 1, and HPLC analysis was performed according to APH stability-indicating analytical method for carbidopa levodopa formulations with Agilent 1100 system.

The HPLC system used herein includes the following components manufactured by Agilent: pump system (model G1311A), diode array detector (model G1315B), autosampler (model G1329A), degasser (model G1379A), thermostat (model G1330B), thermostatted column compartment (model G1316A). The column employed was a new Synergi 4µ Fusion-RP 80A, 250×4.6 mm (Phenomenex®).

HPLC working conditions: wavelength: 280 nm; flow rate: 1.0 ml/min; injection volume: 10 µl; column temperature: 30°C; thermostat temperature: 4°C; stop time: 27 min; pressure: 105 bar.

Mobile phase preparation: Solvent A: acetonitrile, Solvent B: 20 mM potassium dihydrogen phosphate, pH=2.4. The mobile phase B was prepared by weighing 2.72 g/l of potassium dihydrogen phosphate. pH was adjusted to 2.4 by addition of H₃PO₄. Gradient used was according to **Table 2.**

Diluent: 0.1M HCl/MeOH 9:1 (8.3 ml HCl 37% to 1L) --> 0.1 M HCl. STD LDOPA=100.00 mg/100ml. STD CDOPA=25.00 mg/100ml.

Calibration curve is described in Table 3.

**Table 2**

| **Time** | **Solvent A** | **Solvent B** | **Flow** |
|---|---|---|---|
| 0 | 5 | 95 | 1.0 |
| 5 | 5 | 95 | 1.0 |
| 15 | 60 | 40 | 1.0 |
| 20 | 60 | 40 | 1.0 |
| 20.01 | 5 | 95 | 1.2 |
| 27 | 5 | 95 | 1.2 |

**Table 3**

| STD stock solution ^{.} 1000/25 ppm | Volumetric bottle, ml | Final concentration LD/CD ppm |
|---|---|---|
| NA | 10 | 1000/250 |
| 5000 from stock | 10 | 500/125 |
| 5000 from 500/125 | 10 | 250/62.5 |
| 1000 from stock | 10 | 100/25 |
| 5000 from 100/25 | 10 | 50/12.5 |
| 1000 from 100/25 | 10 | 10/2.5 |

One ml of the sample (levodopa/carbidopa formulation) was transferred to a 25 ml amber volumetric glass flask and filled to volume with diluent (0.1 M HCl/MeOH 9/1). The sample was degraded with hydrogen peroxide.

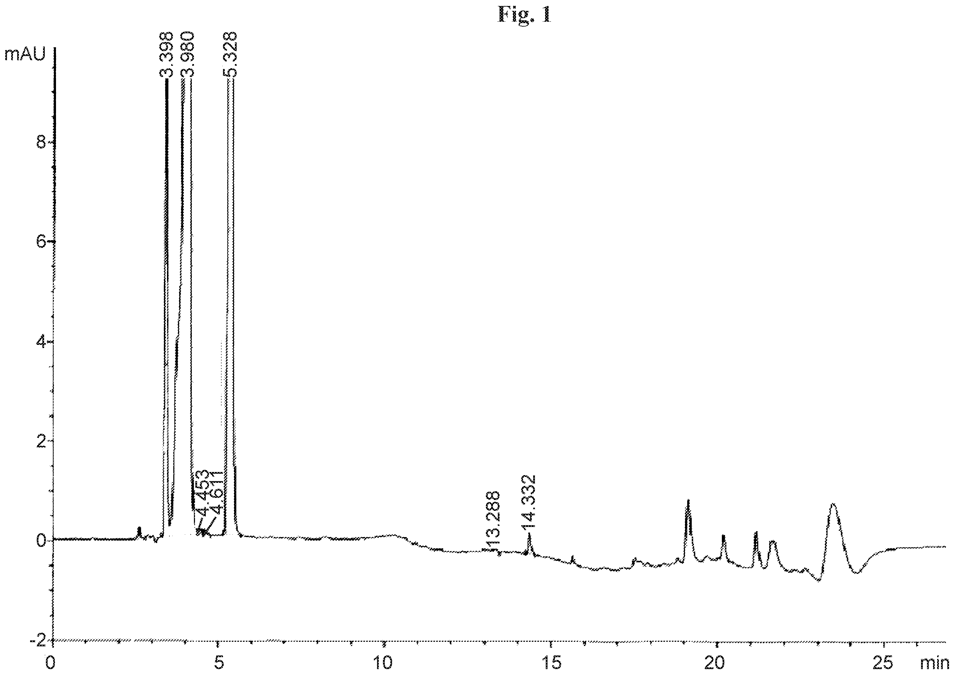

The impurity was observed at retention time of about 14.5±0.2 min (Fig. 1). To ensure that the peak observed is actually the compound of interest, the main degradant peak was collected from analytical HPLC, evaporated under nitrogen stream and reconstituted with diluent. Obtained samples were tested by HPLC/MS.

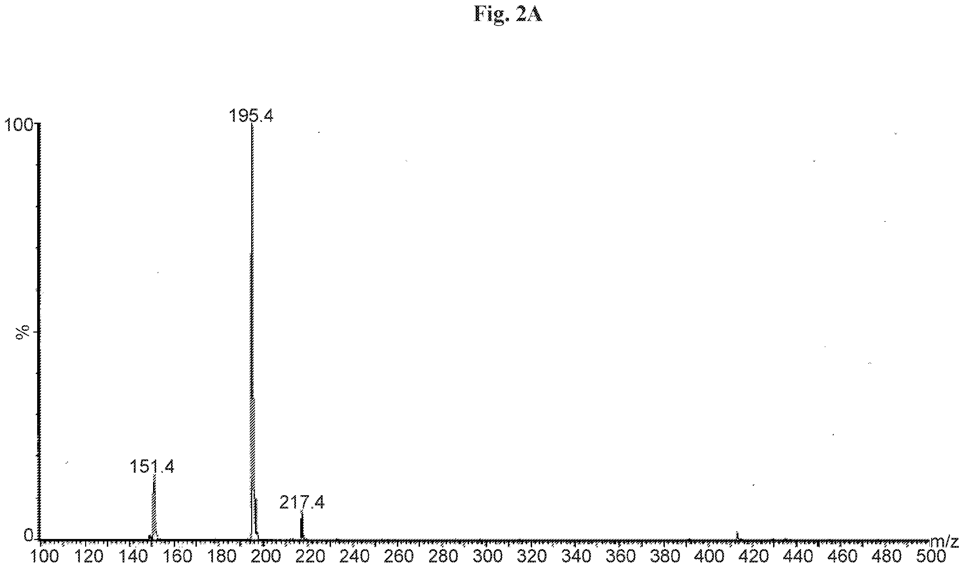

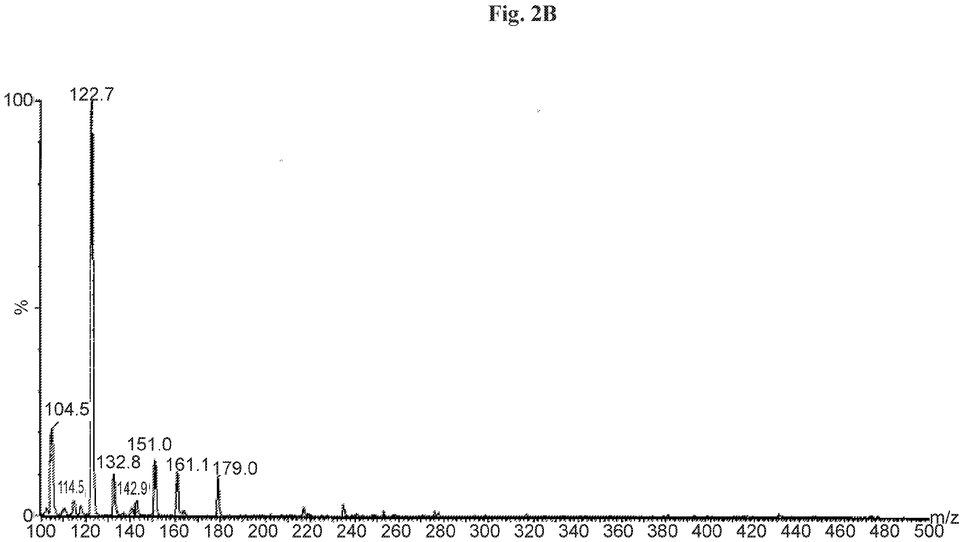

Initially, an MS scan analysis was applied (Figs. 2A-2B). The unknown compound shows clear and intensive signal in negative mode and much nosier signal in positive mode. Therefore it was expected to be more a proton donor than acceptor. The base peak in negative mode was M/Z=195 Da that was suspected as (M-H). The mass difference between this ion and peak M/Z=217 is 22 and that should be the sodium adduct. This is the evidence of the presence of carboxyl or/and phenol groups. Due to this fact, the molecular weight was proposed to be 196 Da.

The peak M/Z=197 (M+H+)⁺ was not found in positive mode, but the peak M/Z=197 (M-H₂O)⁺ is observed. This is typical for oxygen containing molecule.

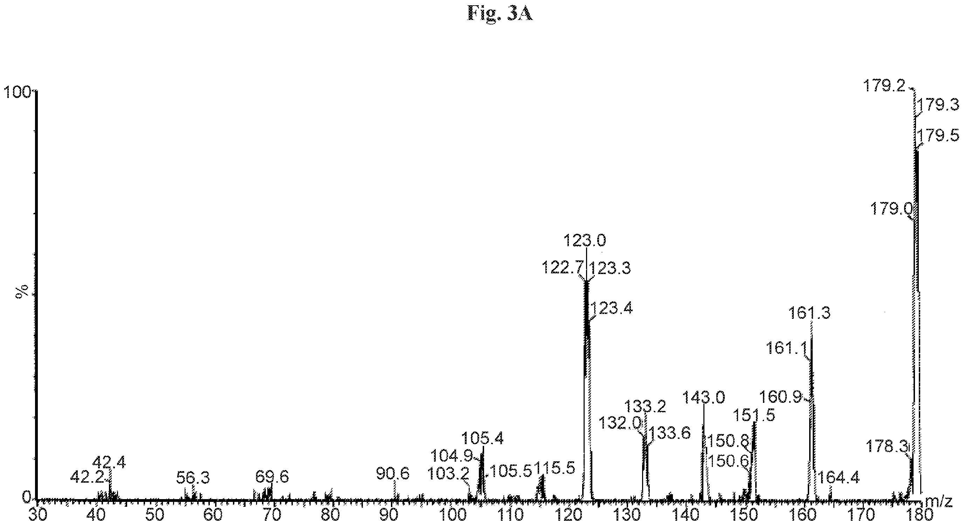

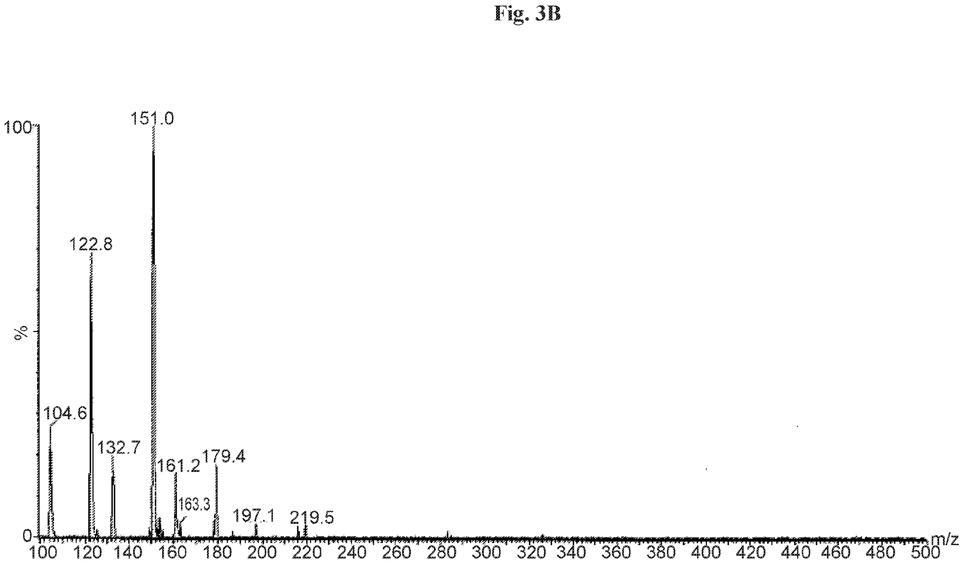

The daughter and parent MS/MS was performed as well to define molecular structure. Peaks observed in positive mode with M/Z=179, 161, 151, 133, 123, 105 were found to be relative. They were defined as in-source fragment ions arising from the molecular ion with M/Z=197. The typical MS and MS/MS spectra are shown on figures (**Figs. 3A-3B**).

The chemical formula of the degradant compound is C₁₀H₁₂O₄, with a molecular structure given by 3-(3,4 dihydroxyphenyl)-2-methylpropanoic acid.

### Example 3. Effect of ascorbic acid with or without EDTA on LD/CD formulation stability

Liquid formulations were prepared by weighing all powders (LD, CD, EDTA, ascorbic acid, and L-Arg) and adding water pre-heated to 73±3°C. The suspension was put in a water bath at 73±3°C and stirred for 10 min until fully dissolved. LD/CD solution was kept at RT for 10 min to cool down. Solutions were divided into glass vials and kept at +25°C and at -20°C for the indicated period of time. Prior to analyses, frozen vials were placed at RT until fully thawed. Formulations were then mixed and subjected to stability analyses. The effect of ascorbic acid with or without EDTA on the stability of LD/CD formulations was measured by HPLC. The levels of the degradant presented in **Tables 4** and **5** (as percentage of the initial CD amount) indicate the level of stability of LD/CD formulations, and suggest that EDTA did not have a significant effect on the stability of LD/CD formulations.

**Table 4**

| LD/CD | L-Arg (%) | Ca-EDTA -Na₂ (%) | Ascorbic acid (%) | t=0 | |
|---|---|---|---|---|---|
| | | | | Degradant | Total* (%) |
| 6/1.4 | 14.80 | 0.2 | 1.0 | 0.13 | 0.76 |
| 6/1.4 | 14.80 | 0 | 1.0 | 0.08 | 0.44 |

**Table 5**

| LD/CD | L-Arg (%) | Ca-EDTA-Na₂ (%) | Ascorbic acid (%) | t=0 | | t=1w (25°) | |
|---|---|---|---|---|---|---|---|
| | | | | Degrandant | Total (%) Total (%) | Degredant | Total (%) |
| 6.0/1.4 | 14.80 | 0.2 | 1.0 | 0.054 | 0.64 | 0.16 | 0.79 |
| 6.0/1.4 | 14.80 | 0 | 1.0 | 0.046 | 0.49 | 0.15 | 0.49 |

| | | | | | | | |
|---|---|---|---|---|---|---|---|
| * percentage of total amount of ingredients. | | | | | | | |

### Example 4. Effect of L-cysteine on the stability of CD/LD containing solutions

Liquid formulations were prepared by weighing all powders (LD, CD, L-cysteine, ascorbic acid, and L-Arg) and adding water pre-heated to 73±3°C. The suspension was put in a water bath at 73±3°C and stirred for 10 min until fully dissolved. LD/CD solution was kept at RT for 10 min to cool down. For CD formulations, CD, L-cysteine, and ascorbic acid were weighed, and pre-heated water (60°C) was added. Solutions were divided into glass vials and kept at +25°C and at -20°C for the indicated period of time. Prior to analyses, frozen vials were placed at RT until fully thawed. Formulations were then mixed and subjected to stability analyses. The effect of L-cysteine on the stability of carbidopa formulations when stored at 25°C, either exposed to air or maintained under anaerobic conditions (N₂), was analyzed using HPLC. The levels of the degradant presented in Table 6 (as percentage of the initial CD amount) point toward the level of stability of carbidopa formulations.

As indicated in Table 6, ascorbic acid with 0.1% L-cysteine was sufficient to inhibit degradant formation in formulations containing carbidopa (with or without levodopa) when kept under anaerobic conditions at 25°C for at least 5 weeks. As may further be deduced from this Table, L-cysteine reduced degradant formation under aerobic conditions at 25°C in a dose-dependent manner.

In formulations containing carbidopa and 0.4% L-cysteine, degradant formation was inhibited during the preparation of the formulation. These formulations were stable for at least 5 weeks at 25°C under both aerobic and anaerobic conditions. Formulations containing carbidopa are more stable when they also contain LD upon exposure to air.

**Table 6**

| LD/CD | L-Arg | Ascorbic acid (%) | **L-Cysteine (%)** | | Degradant | | |
|---|---|---|---|---|---|---|---|
| | | | | | t=0 | t=5 days | t=5 weeks |
| 6/1.4 | 14.8 | 0.5 | **0.1** | N₂ | 0.08 | 0.22 | 0.28 |
| | | | | **O₂** | | **0.59** | 0.77 |
| | | 0.5 | **0.2** | N₂ | 0.08 | 0.20 | 0.25 |
| | | | | **O**₂ | | **0.36** | **0.32** |
| | | 0.5 | **0.4** | N₂ | 0.00 | 0.14 | 0.14 |
| | | | | **O**₂ | | **0.14** | **0.17** |
| 0/4 | 4.6 | 0.5 | **0.1** | N₂ | | 0.16 | |
| | | | | **O**₂ | | **1.42** | |
| | | 0.5 | **0.2** | N₂ | | 0.16 | |
| | | | | **O**₂ | | **0.56** | |
| | | 0.5 | **0.4** | N₂ | 0.04 | 0.15 | |
| | | | | **O**₂ | | **0.35** | |

### Example 5. Effect of L-cysteine on 6/1.4% LD/CD formulation stability

Liquid formulations were prepared as described in Example 3. The effect of L-cysteine on the stability of 6/1.4% levodopa/carbidopa solutions at 25°C was analyzed using HPLC. The levels of the degradant presented in **Tables 7-10** (as percentage of the initial CD amount) point toward the level of stability of the referenced formulations.

The results show that levodopa/carbidopa formulations were more stable with both ascorbic acid and L-cysteine, as compared to ascorbic acid alone, suggesting that L-cysteine and ascorbic acid have a synergistic effect in preventing degradant formation. Other results showed that L-cysteine alone had no effect at all (data not shown). Furthermore, L-cysteine inhibited degradant formation during formulation preparation and maintained the stability of the formulation for at least up to 5 weeks at 25°C, in a dose dependent manner. Increasing the amount of ascorbic acid reduces degradant formation, but this was significantly less efficient than the combination of ascorbic acid with L-Cys.

**Table 7**

| LC/CD (%) | L-Arg (%) | Ascorbic acid (%) | L-Cys (%) | Degradant |
|---|---|---|---|---|
| | | | | t=0 |
| 6/1.4 | 14.8 | 0.5 | 0.2 | 0.02 |
| | | 0.75 | 0.0 | 0.16 |

**Table 8**

| LC/CD (%) | L-Arg (%) | Ascibic acid (%) | **L-Cys (%)** | Degradant | | |
|---|---|---|---|---|---|---|
| | | | | t=0 | t=5 d | t=5 wk |
| 6/1.4 | 14.8 | 0.75 | **0** | **0.49** | **0.93** | **1.08** |
| | | | 0.1 | 0.10 | 0.35 | 0.32 |
| | | 0.50 | 0.1 | 0.16 | 0.28 | 0.45 |
| | | | 0.4 | 0.00 | 0.15 | 0.13 |

**Table 9**

| LD/CD (%) | L-Arg (%) | Ascorbic acid (%) | **L-Cys (%)** | Degradant | | |
|---|---|---|---|---|---|---|
| | | | | t=0 | t=1 d | t=4 d |
| 6/1.4 | 14.8 | 0.5 | 0.4 | 0.00 | 0.00 | 0.27 |
| | | | 0.1 | 0.25 | 0.69 | 1.26 |
| | | 0.75 | 0.1 | 0.23 | 0.56 | 0.85 |
| | 15.1 | 1.0 | 0.2 | 0.00 | 0.26 | 0.51 |

**Table 10**

| LD/CD (%) | L-Arg (%) | Ascorbie acid (%) | **L-Cys (%)** | Degradant | | | |
|---|---|---|---|---|---|---|---|
| | | | | t=0 | t=10 d | t=3 weeks | t=3 months |
| 6/1.4 | 14.8 | 0.5 | 0.1 | 0.15 | 0.43 | 0.46 | 0.87 |
| | | 0.75 | 0.0 | 1.01 | 1.40 | 1.37 | 2.00 |

### Example 6. Effect of Tween-80 and Na-ascorbate on LD/CD formulation stability

Liquid formulations were prepared by weighing all powders (LD, CD, L-cysteine, ascorbic acid, Na-ascorbate and L-Arg) and adding water pre-heated to 73±3°C. The suspension was put in a water bath at 73±3°C and stirred for 10 min until fully dissolved. LD/CD solution was kept at RT for 10 min to cool down. Then, Tween-80 was added. Solutions were divided in to glass vials and kept at +25°C and at -20°C for the indicated period of time. Prior to analyses, frozen vials were placed at RT until fully thawed. Formulations were then mixed and subjected to stability analyses. The effect of Tween-80 and Na-ascorbate on the stability of carbidopa/levodopa formulations was analyzed using HPLC. The levels of the degradant presented in **Table 11** (as percentage of the initial CD amount) point toward the level of stability of carbidopa/levodopa formulations.

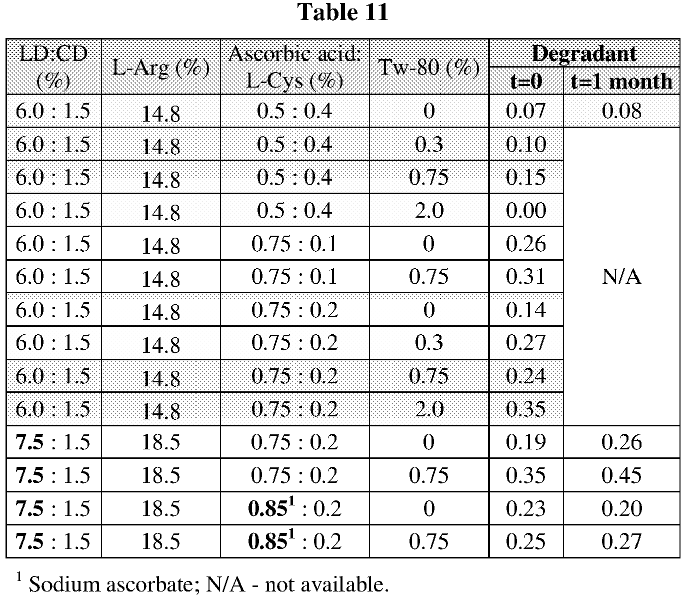

The results demonstrate that Tween-80 did not have an effect on degradant formation. It is also shown that the effect of L-cysteine on the stability of the formulations was dose dependent. As further shown, the effect of Na-ascorbate and ascorbic acid on the stability of the formulations and degradant formation was similar.

### Example 7. Effect of ascorbic acid with or without L-Cys or NAC on long term stability of LD/CD formulations

Liquid formulations were prepared by weighing all powders (LD, CD, arginine, L-cysteine or NAC, and ascorbic acid or Na-ascorbate) and by adding water pre-heated to 73±3°C. The suspension was put in a water bath at 73±3°C and stirred until fully dissolved. LD/CD solution was kept at RT to cool down. Then Tween-80 was added. Solutions were divided into glass vials and kept at +25°C and at -20°C for the indicated period of time. Prior to analyses, frozen vials were placed at RT until fully thawed. Formulations were then mixed and subjected to stability analyses. The effect of ascorbic acid with or without L-cysteine or NAC on the stability of carbidopa/levodopa formulations was analyzed using HPLC. The levels of the degradant presented in Table 12 (as percentage of the initial CD amount) indicates the level of stability of carbidopa/levodopa formulations.

The results shown in **Table 12** suggest that formulations containing both ascorbic acid and NAC are more stable than formulations having only ascorbic acid, at a) T₀, i.e., immediately following formulation preparation, b) for at least 9 months at -20°C, and c) at for least 1 month at ambient temperature.

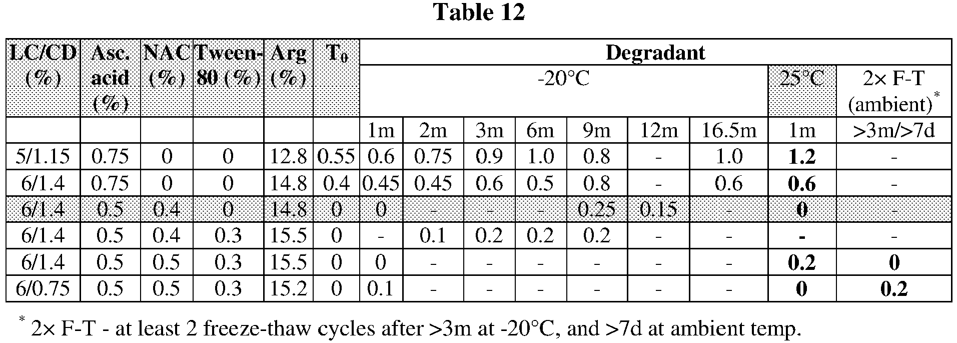

### Example 8. Effect of antioxidants on the stability of CD formulations

Liquid formulations with carbidopa and arginine were prepared as described above. The effect of antioxidants on the stability of carbidopa formulations was analyzed using HPLC. The levels of the degradant presented in **Tables 13** and **14** (as percentage of the initial CD amount) point toward the level of stability of carbidopa formulations.

The results in **Tables 13** and **14** suggest that formulations containing ascorbic acid + L-cysteine were significantly more stable than the formulation containing Na-bisulfite (formulations 3 & 4 vs. formulations 1 & 2). The same amount of impurities were measured with 0.075 and 0.1% Na-bisulfite, suggesting that the maximum possible protection with Na-bisulfite was attained.

In sum, the combination of ascorbic acid/L-cysteine is able to prevent degradant and other impurities formation, such as hydrazine (see other examples), while Na-bisulfite does not protect formulations containing carbidopa to the same extent.

**Table 13**

| **7 weeks at (-20°C)** | 1 | | | 4 |
|---|---|---|---|---|
| | 0.1% Na Bisulfite | 2 0.075% Na Bisulfite | 3 0.4% Asc. + 0.2% L-Cys | 0.5% Asc. + 0.1% L-Cys |
| Methyl-Dopa | 0.15 | 0.15 | 0.15 | 0.15 |
| Unknown 2 | 0.18 | 0.17 | 0 | **0** |
| Degradant | 0.54 | 0.55 | **0** | 0.16 |
| **Sum of all Impurities** | **1.14** | **1.15** | **0.44** | **0.65** |

**Table 14**

| | | Peak 14.3 min (degradant) | | |
|---|---|---|---|---|
| | | t=0 | **t=3 weeks 2-8°C** | t=3 weeks 25°C |
| 2 | 4% CD - 3.7% L-Arg 0.075% sodium bisulfite | 0.30 | **1.12 ± 0.19** | 1.08 ± 0.07 |
| 3 | 4% CD - 4.62% L-Arg 0.4% ascorbic + 0.2% L-cysteine | 0.06 | **0.15 ± 0.01** | 0.29 ± 0.07 |
| 4 | 4% CD - 4.62% L-Arg 0.5% ascorbic + 0.1% L-cysteine | 0.11 | **0.44 ± 0.21** | 0.63 ± 0.39 |

### Example 9. Effect of various antioxidants and different concentrations of arginine on the stability of formulations containing 4% CD

Liquid formulations with carbidopa and arginine (**Table 15**) were prepared as described above. The effect various antioxidants and different concentrations of arginine on the stability of formulations containing 4% CD, and stored under aerobic (air) or anaerobic (N₂) conditions, at ambient (25°C) or cold (2-8°C) temperature was evaluated using HPLC analysis. The levels of the degradant and total impurities as presented in **Tables 16** and **17**, respectively, point toward the level of stability of carbidopa formulations.

The results as presented in **Tables 16** and **17** indicate that formulations containing more arginine were more stable when exposed to air at 25°C (formulations 2 *vs.* 3). Further, formulations containing Na-bisulfite were less stable than the formulation containing ascorbic acid and L-cysteine (formulations 2 vs. 1, respectively) when stored under nitrogen (anaerobic conditions). N₂ provided significant protection from degradation and degradant formation. Formulations exposed to air were more stable when kept refrigerated, as compared to room (ambient) temperature.

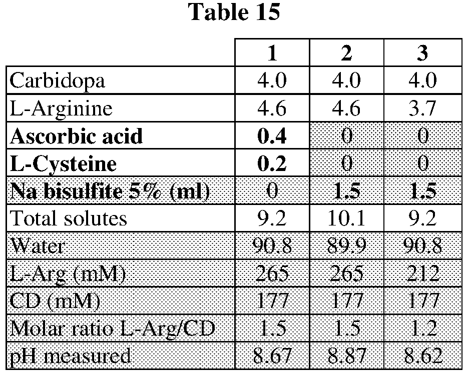

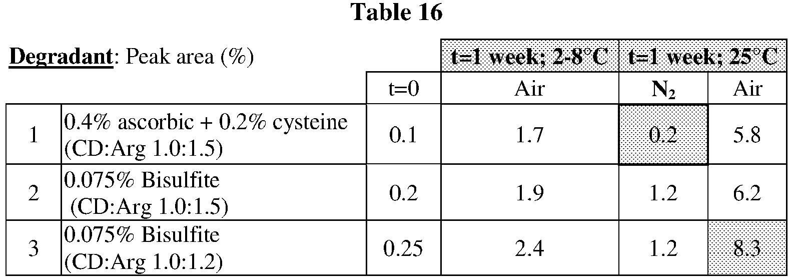

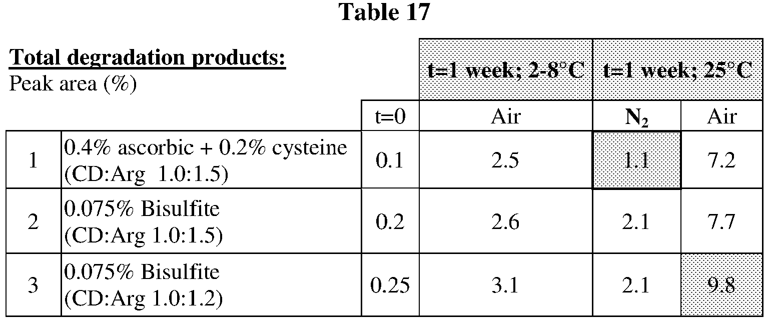

### Example 10. Effect of various antioxidants on the stability of formulations containing 4% CD at 40°C

Liquid formulations with carbidopa and arginine (**Table 18**) were prepared as described above. The effect various antioxidants on the stability of formulations containing 4% CD at 40°C was evaluated using HPLC analysis. The levels of the degradant (as percentage of the initial CD amount) and total impurities, presented in **Tables 19** and **20**, respectively, indicate the level of stability of carbidopa formulations.

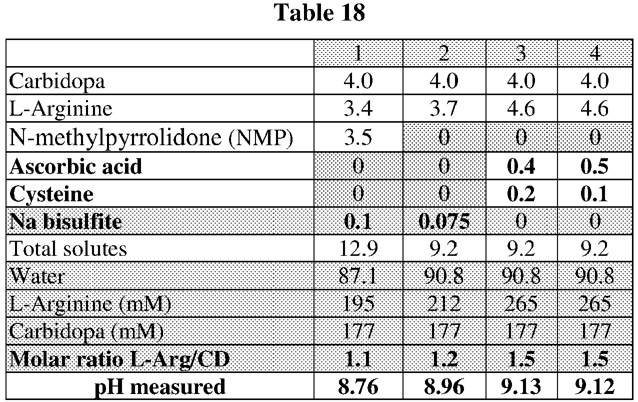

The results presented in **Tables 19-20** suggest that formulations containing Na-bisulfite were less stable than the formulations containing ascorbic acid and L-cysteine (formulations 1&2 *vs.* 3&4), both during preparation and when stored at 40°C. Moreover, there was a cysteine dose-response, i.e., the higher the concentration of L-cysteine, the less degradant was formed. No dose response was observed with Na-bisulfite, suggesting that the maximum possible protection may be attained with 0.075% Na-bisulfite.

**Table 19 - Degradant**

| | | t=0 | t= 2d/40°C | t= 5d/40°C |
|---|---|---|---|---|
| 1 | 4% CD - 3.4% L-Arg + 3.5% NMP 0.1% sodium bisulfite | 0.27 ± 0.01 | 1.52 ± 0.56 | 2.60 ± 0.67 |
| 2 | 4% CD - 3.7% L-Arg 0.075% sodium bisulfite | 0.30 ± 0.01 | 1.41 ± 0.54 | 2.95 ± 0.60 |
| 3 | 4% CD - 4.62% L-Arg 0.4% ascorbic + 0.2% L-Cys | 0.06 ± 0.01 | 0.38 ± 0.14 | 1.26 ± 0.45 |
| 4 | 4% CD - 4.62% L-Arg 0.5% ascorbic + 0.1% L-Cys | 0.11 ± 0.01 | 0.50 ± 0.23 | 1.97 ± 0.52 |

**Table 20 - Total impurities**

| | | t=0 | t= 2d/40°C | t= 5d/40°C |
|---|---|---|---|---|
| 1 | 4% CD - 3.4% L-Arg + 3.5% NMP 0.1% sodium bisulfite | 0.61 | 2.00 ± 0.54 | 3.19 ± 0.65 |
| 2 | 4% CD - 3.7% L-Arg 0.075% sodium bisulfite | 0.60 | 1.86 ± 0.56 | 3.61 ± 0.60 |
| 3 | 4% CD - 4.62% L-Arg 0.4% ascorbic + 0.2% L-Cys | 0.29 | 0.92 ± 0.16 | 2.15 ± 0.59 |
| 4 | 4% CD - 4.62% L-Arg 0.5% ascorbic + 0.1% L-Cys | 0.34 | 1.17 ± 0.28 | 2.82 ± 0.61 |

### Example 11. Effect of ascorbic acid combined with various antioxidants on the stability of formulations containing 4% CD

Liquid formulations with carbidopa and ascorbic acid with or without additional antioxidants were prepared as described above. The combination effect between ascorbic acid and various antioxidants on the stability of formulations containing 4% CD at 25°C was evaluated using HPLC analysis. The levels of the degradant (as percentage of the initial CD amount) and total impurities presented in **Tables 21** and **22** respectively, point toward the level of stability of carbidopa formulations.

The results presented in **Tables 21-22** show that ascorbic acid, 0.5%, was insufficient for the prevention of degradant formation in a formulation containing carbidopa. Furthermore, ascorbic acid requires another antioxidant in order to exert its maximum antioxidant activity, e.g., ascorbic acid, 0.5%, and L-cysteine, NAC, or Na-bisulfite inhibited carbidopa degradation in a synergistic manner. Formulations containing ascorbic acid and L-cysteine had the lowest amount of degradant after 3 days at 25°C.

The effect of Na-bisulfite on carbidopa degradation was similar to that obtained with no antioxidants at all.

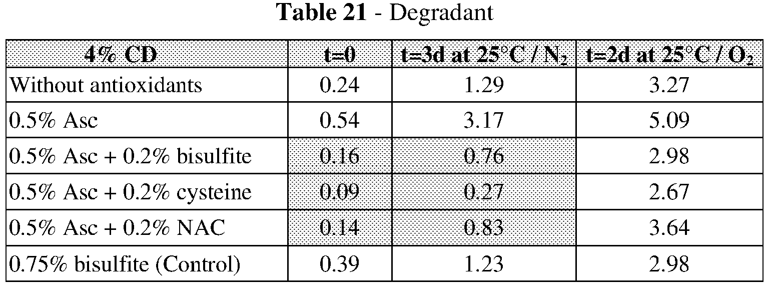

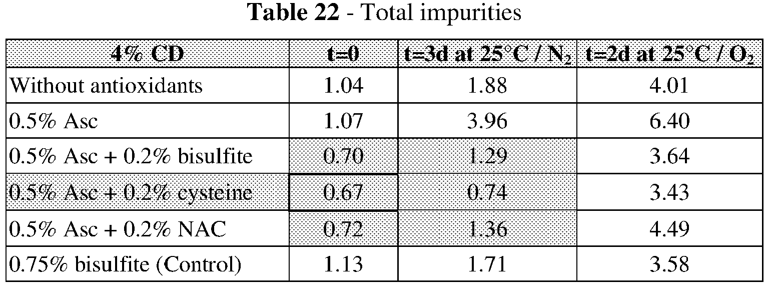

### Example 12. Effect of antioxidants on the stability of formulations containing 4% CD at 25°C

Liquid formulations with carbidopa (4%) and arginine (**Table 23**) were prepared as described above, and the effect of various antioxidants on the stability of those formulations at 25°C was evaluated using HPLC analysis. The levels of the degradant (as percentage of the initial CD amount) and total impurities presented in **Table 24** indicate the level of stability of those formulations.

The results presented in **Table 24** suggest that ascorbic acid, bisulfite, or cysteine, each used alone, did not inhibit degradant formation. Combinations between bisulfite and cysteine or ascorbic acid did not inhibit degradant formation. There was a synergistic inhibitory effect on degradant formation between ascorbic acid and cysteine, but no such synergism between cysteine and bisulfite was observed. Such synergistic effects can be seen between ascorbic acid and bisulfite (with higher ascorbic acid concentrations). These results further suggest that formulations containing the unique combination of ascorbic acid and cysteine may provide the best means for the inhibition of degradant formation.

Ascorbic acid, at 0.2%, was not sufficient for the prevention of degradant formation. With 0.2% cysteine, 0.5% ascorbic acid was more effective than 0.2% in reducing the total amount of impurities and degradant formation, suggesting that at least 0.5% ascorbic acid with 0.2% cysteine is desirable.

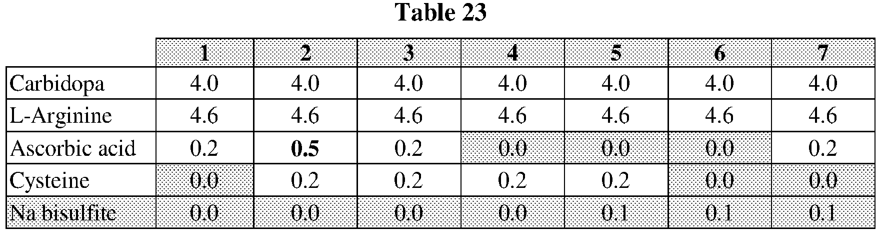

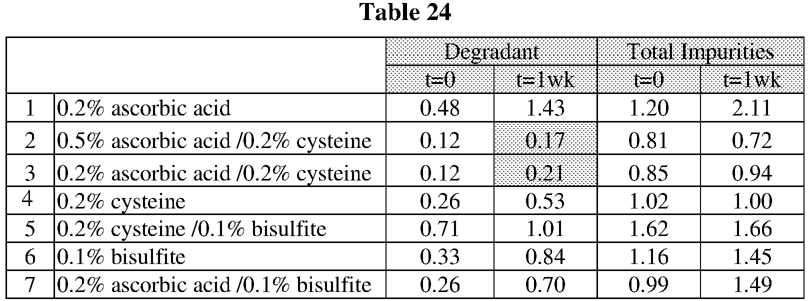

### Example 13. Determination of the level of hydrazine in CD and CD/LD formulations

The determination of hydrazine was carried out by derivatization using Aceton-d6. The hydrazine derivative was analyzed by gas chromatography mass spectrometry (GC/MS). The specific mass of hydrazine derivative was measured in the selected ion monitoring mode (SIM-mode) according to Solvias standard operating procedures (SOP's).

Liquid formulations with carbidopa, levodopa, and arginine **(Table 25**) were prepared as described above. Formulations 7, 9-14 and 18 are according to the invention. The levels of hydrazine in the referenced formulations were measured **(Table 26**).

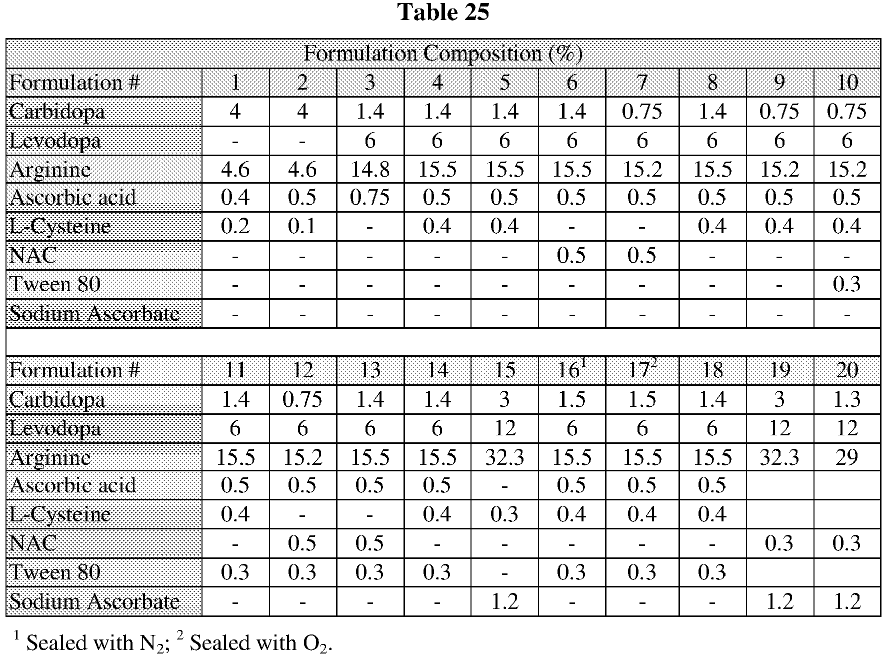

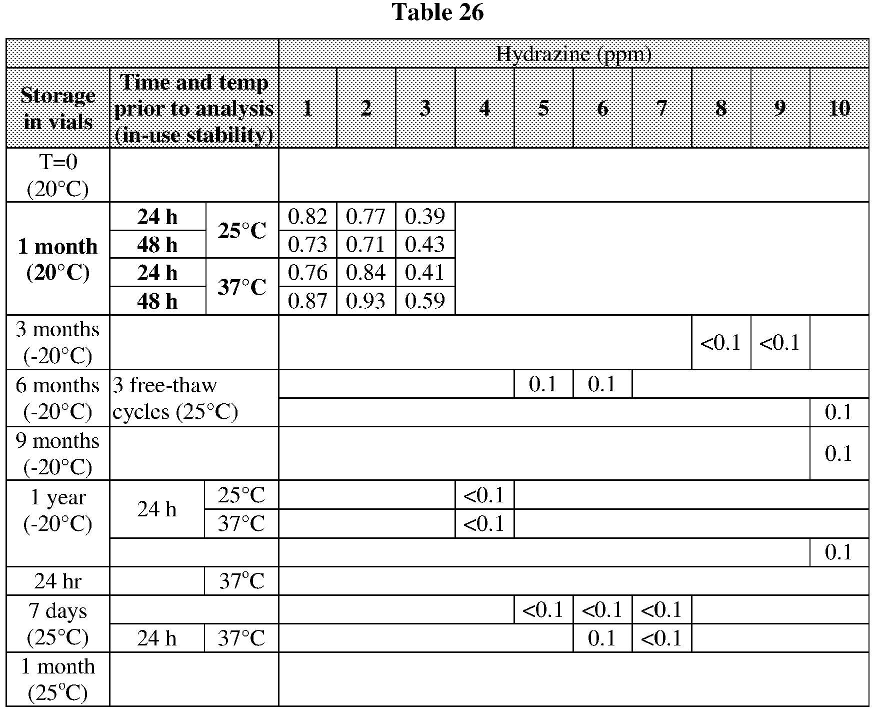

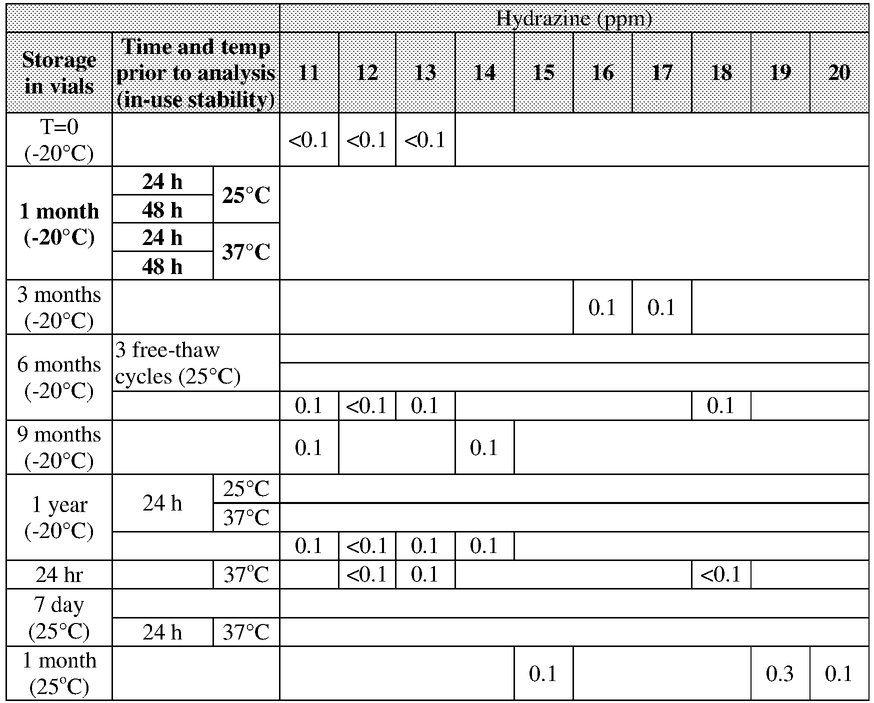

The results presented in **Table 26** clearly show that the levels of hydrazine were at least 2 fold lower in levodopa formulations vs. formulations without levodopa. Furthermore, formulations comprising L-cysteine or NAC showed at least 4-fold lower levels of hydrazine compared to formulations without L-cysteine or NAC.

### Example 14. CD/LD formulations

Based on the discoveries of combinations that have reduced degradant and hydrazine formation, we have developed new CD/LD formulations. These formulations are shown in **Tables 27** (according to the invention) and **28** (not according to the invention).

**Table 27**

| **DS (%)** | **LD** | **CD** | **Arginine** | **Ascorbic acid** | **L-Cysteine** | **NAC** | **Tween-80** | **pH** |
|---|---|---|---|---|---|---|---|---|
| 1 | 6 | 1.4 | 15.5 | 0.5 | 0.4 | - | 0.3 | 9.4-9.6 |
| 2 | 6 | 1.4 | 15.5 | 0.5 | - | 0.5 | 0.3 | 9.4-9.6 |
| 3 | 6 | 0.75 | 15.2 | 0.5 | 0.4 | - | 0.3 | 9.4-9.6 |
| 4 | 6 | 0.75 | 15.2 | 0.5 | - | 0.5 | 0.3 | 9.4-9.6 |
| Margins | 6 | 0.6-1.4 | 15-16 | 0.5 | 0.4 | 0.5 | 0.3 | 9.4-9.6 |

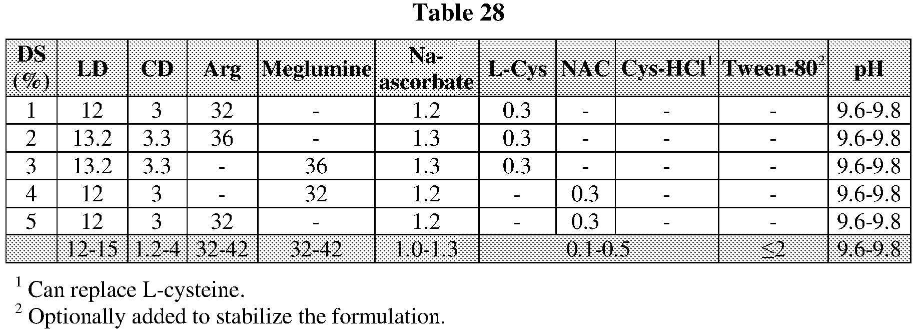

Additional formulations that may be used in the context of those disclosed herein are provided in **Table 29.** The formulations may include additional components (e.g., any of those described herein). **Tables 30** and **31** describe further formulations that can be used in the context of those described herein.

**Table 29**

| LD conc. (%) | CD conc. (%) | Amino acid (conc. %) | Other (conc %) |
|---|---|---|---|
| 4.8 | 1.4 | Arg (11.0) | |
| 4.8 | 1.4 | Arg (12.1) | |
| 4.8 | 1.4 | Arg (12.7) | |
| 5.4 | 1.5 | Arg (13.5) | |
| 5.4 | 1.5 | Arg (14.8) | |
| 6 | 1.5 | Arg (14.8) | |
| 6 | 1.5 | Arg (16.0) | |
| 7 | 2 | Arg (17.8) | |
| 7 | 1.5 | Arg (14.1) | Dextrose (5.0) |
| 8 | 1.5 | Arg (15.7) | Dextrose (5.0) |
| 10 | 1.5 | Arg (19.2) | Dextrose (5.0) |
| 6 | 1.5 | Arg (9.3) | NaOH (4.6) |
| 8 | 1.5 | Arg (15.7) | Meglumine (3.2) |
| 8 | 1.5 | Arg (12.2) | Meglumine (7.9) |
| 10 | 1.5 | Arg (19.2) | Meglumine (4.0) |
| 10 | 1.5 | Arg (14.6) | Meglumine (9.9) |
| 7 | 1.5 | Arg (14.1) | Meglumine (2.8) |
| 7 | 1.5 | Arg (10.7) | Meglumine (6.9) |
| 6 | 1.5 | Arg (13.5) | Na-Asc (1.0) |
| 6 | 1.5 | Arg (14.2) | Na-Asc (1.0) |
| 6 | 1.5 | Arg (14.8) | Na-Asc (1.0) |
| 6 | 1.5 | Arg (16.0) | Na-Asc (1.0) |
| 4.8 | 1.4 | Arg (11.0) | Na-Asc (1.0) |
| 4.8 | 1.4 | Arg (11.6) | Na-Asc (1.0) |
| 4.8 | 1.4 | Arg (12.1) | Na-Asc (1.0) |
| 4.8 | 1.4 | Arg (12.7) | Na-Asc (1.0) |
| 6 | 1.5 | Arg (14.8) | Asc (1.0) |
| 6 | 1.5 | Arg (15.8) | Na-Asc (1.0) |
| 6 | 1.5 | Arg (15.8) | Asc (1.0) |
| 6 | 1.5 | Arg (16.8) | Na-Asc (1.0) |
| 6 | 1.5 | Arg (16.8) | Asc (1.0) |
| 5.4 | 1.5 | Arg (12.3) | Na-Asc (1.0) |
| 5.4 | 1.5 | Arg (12.3) | Asc (1.0) |
| 5.4 | 1.5 | Arg (13.5) | Na-Asc (1.0) |
| 5.4 | 1.5 | Arg (13.5) | Asc (1.0) |
| 5.4 | 1.5 | Arg (14.8) | Na-Asc (1.0) |
| 5.4 | 1.5 | Arg (14.8) | Asc (1.0) |
| 6 | 1.5 | Arg (16.0) | Asc(1.0) |
| 7 | 2 | Arg (17.8) | Asc (1.0) |
| 7 | 2 | Arg (17.8) | Na-Asc (1.0) |
| 12 | 3 | Arg (24.4) | |
| 12 | 3 | Arg (29.6) | |
| 12 | 3 | Arg (32.1) | |
| 7 | 0.5 | Arg | |
| 7 | 1 | Arg | |
| 7 | 1.5 | Arg | |
| 7 | 2 | Arg | |
| 6 | 0.5 | Arg (14.2) | |
| 6 | 1 | Arg (14.8) | |
| 6 | 2 | Arg (16.5) | |
| 0 | 2 | | |

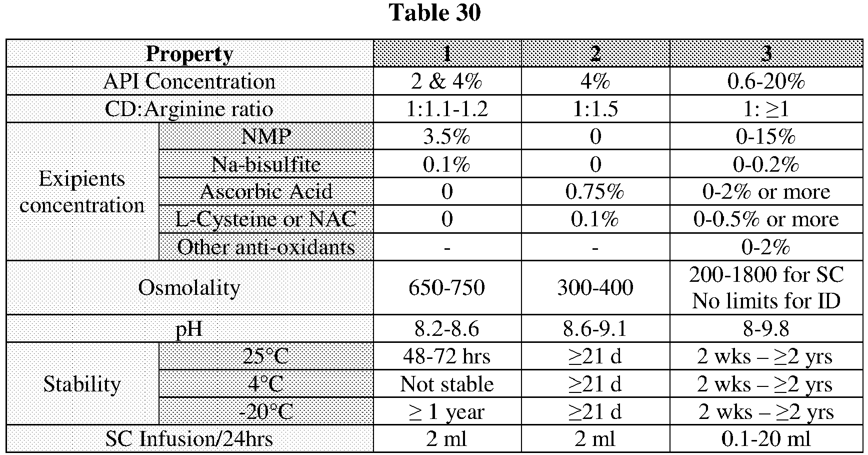

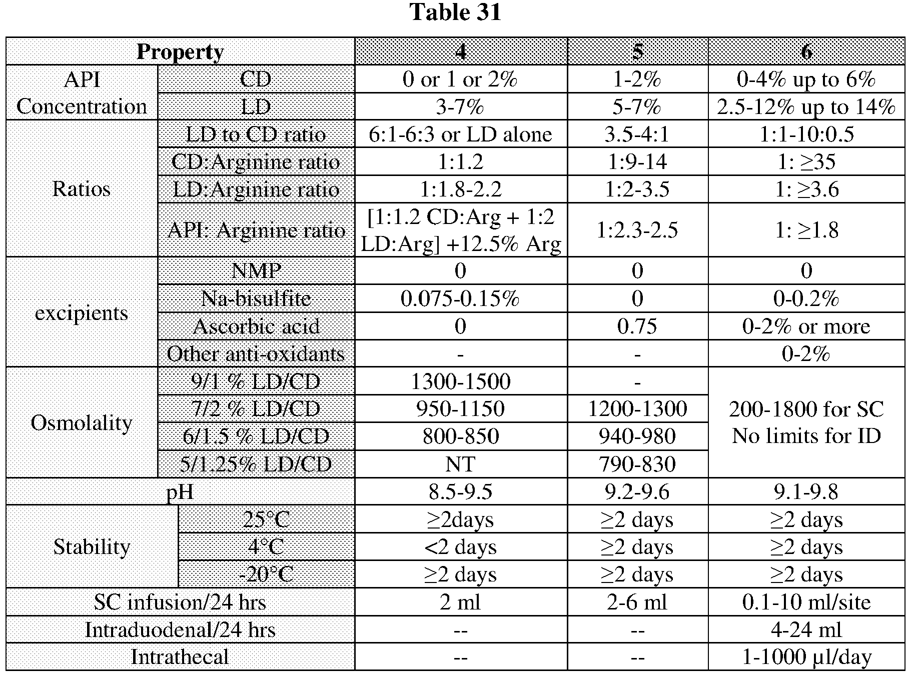

The formulations in Tables 29-31 are not according to the invention.

Unless otherwise indicated, all numbers expressing quantities of ingredients, reaction conditions, and so forth used in the specification and claims are to be understood as being modified in all instances by the term "about". Accordingly, unless indicated to the contrary, the numerical parameters set forth in this specification and attached claims are approximations that may vary depending upon the desired properties sought to be obtained by the present invention.

## Claims

1. A pharmaceutically acceptable liquid composition comprising:
about 0.75% by weight carbidopa;
about 6% by weight levodopa;
about 12% to about 36% by weight arginine;
about 0.5% by weight ascorbic acid or a salt thereof;
about 0.1% to about 0.6% by weight L-cysteine, or a salt thereof, or about 0.01% to about 1% by weight N-acetylcysteine (NAC); and
optionally, about 0.1% to about 0.5% by weight, preferably about 0.3% by weight, polysorbate 80,
wherein said composition, after 1-24 hours at 25°C, comprises less than 0.1 µg/ml hydrazine, as determined by a GCMS method.

2. The composition of claim 1, wherein the composition has less than 1% by weight 3,4-dihydroxyphenyl-2-methylpropionic acid (degradant), relative to the amount of carbidopa, as determined by HPLC.

3. The composition of claim 1, wherein said salt of ascorbic acid is sodium ascorbate, potassium ascorbate or calcium ascorbate.

4. The composition of claim 1, comprising about 15.2% by weight arginine, about 0.5% by weight ascorbic acid, about 0.4% by weight L-cysteine and about 0.3% by weight polysorbate 80.

5. The composition of claim 1, comprising about 15.2% by weight arginine, about 0.5% by weight ascorbic acid, about 0.5% by weight NAC, and about 0.3% by weight polysorbate 80.

6. The composition of claim 1, comprising 6% by weight levodopa, 0.75% by weight carbidopa, 15.2% by weight arginine, 0.5% by weight ascorbic acid, and 0.5% by weight NAC.

7. A pharmaceutically acceptable liquid composition comprising:
about 0.6% to 1.4% by weight carbidopa;
about 6% by weight levodopa;
about 15% to about 16% by weight arginine;
about 0.5% by weight ascorbic acid or a salt thereof;
about 0.4% by weight L-cysteine or about 0.5% by weight N-acetylcysteine (NAC); and
about 0.3% by weight polysorbate 80,
wherein said composition, after 1-24 hours at 25°C, comprises less than 0.1 µg/ml hydrazine, as determined by a GCMS method.

8. The composition of any one of claims 1 to 7, comprising less than 0.1 µg/ml hydrazine after 1-30 days, at a temperature of 25°C, as determined by GCMS.

9. A pharmaceutically acceptable liquid composition according to any one of claims 1 to 8, for use in treating a disease, disorder or condition associated with loss of dopamine or dopaminergic neurons.

10. The composition for use of claim 9, where said disease, disorder or condition is Parkinson's disease.

11. The composition for use of claim 9 or 10, wherein the composition is administered substantially continuously.

12. The composition for use of any one of claims 9 to 11, wherein the composition is administered subcutaneously.

13. The composition for use of any one of claims 9 to 12, wherein the composition is administered to a human.

14. The composition for use of any one of claims 9 to 13, wherein the composition is administered at a rate of 0.01 ml/hour/site to 0.4 ml/hour/site.

15. The composition for use of claim 14, wherein the composition is administered at a rate of 0.16 ml/hour/site to 0.24 ml/hour/site.

16. The composition for use of any one of claims 9 to 11, wherein the composition is administered intraduodenally at a rate of 1.0 ml/hour during the daytime and at a rate of 0 ml/hour to 0.5 ml/hour at night.

## Patentansprüche

1. Pharmazeutisch akzeptable flüssige Zusammensetzung, umfassend:
etwa 0,75 Gew.-% Carbidopa;
etwa 6 Gew.-% Levodopa;
etwa 12 bis etwa 36 Gew.-% Arginin;
etwa 0,5 Gew.-% Ascorbinsäure oder ein Salz davon;
etwa 0,1 bis etwa 0,6 Gew.-% L-Cystein oder ein Salz davon oder etwa 0,01 bis etwa 1 Gew.-% N-Acetylcystein (NAC); und gegebenenfalls etwa 0,1 bis etwa 0,5 Gew.-%, vorzugsweise etwa 0,3 Gew.-% Polysorbat 80,
worin die Zusammensetzung nach 1-24 Stunden bei 25 °C weniger als 0,1 µg/ml Hydrazin umfasst, wie durch eine GCMS-Methode bestimmt.

2. Zusammensetzung gemäß Anspruch 1, worin die Zusammensetzung weniger als 1 Gew.-% 3,4-Dihydroxyphenyl-2-methylpropionsäure (Abbauprodukt) aufweist, bezogen auf die Menge von Carbidopa, wie durch HPLC bestimmt.

3. Zusammensetzung gemäß Anspruch 1, worin das Salz der Ascorbinsäure Natriumascorbat, Kaliumascorbat oder Calciumascorbat ist.

4. Zusammensetzung gemäß Anspruch 1, umfassend etwa 15,2 Gew.-% Arginin, etwa 0,5 Gew.-% Ascorbinsäure, etwa 0,4 Gew.-% L-Cystein und etwa 0,3 Gew.-% Polysorbat 80.

5. Zusammensetzung gemäß Anspruch 1, umfassend etwa 15,2 Gew.-% Arginin, etwa 0,5 Gew.-% Ascorbinsäure, etwa 0,5 Gew.-% NAC und etwa 0,3 Gew.-% Polysorbat 80.

6. Zusammensetzung gemäß Anspruch 1, umfassend 6 Gew.-% Levodopa, 0,75 Gew.-% Carbidopa, 15,2 Gew.-% Arginin, 0,5 Gew.-% Ascorbinsäure und 0,5 Gew.-% NAC.

7. Pharmazeutisch akzeptable flüssige Zusammensetzung, umfassend:
etwa 0,6 bis 1,4 Gew.-% Carbidopa;
etwa 6 Gew.-% Levodopa,
etwa 15 bis etwa 16 Gew.-% Arginin,
etwa 0,5 Gew.-% Ascorbinsäure oder ein Salz davon,
etwa 0,4 Gew.-% L-Cystein oder etwa 0,5 Gew.-% N-Acetylcystein (NAC); und
etwa 0,3 Gewichtsprozent Polysorbat 80,
worin die Zusammensetzung nach 1-24 Stunden bei 25 °C weniger als 0,1 µg/ml Hydrazin umfasst, wie durch eine GCMS-Methode bestimmt.

8. Zusammensetzung gemäß einem beliebigem der Ansprüche 1 bis 7, umfassend weniger als 0,1 µg/ml Hydrazin nach 1-30 Tagen bei einer Temperatur von 25 °C, wie durch GCMS bestimmt.

9. Pharmazeutisch akzeptable flüssige Zusammensetzung gemäß einem beliebigem der Ansprüche 1 bis 8 zur Verwendung bei der Behandlung einer Erkrankung, einer Störung oder eines Zustandes im Zusammenhang mit dem Verlust von Dopamin oder dopaminergen Neuronen.

10. Zusammensetzung zur Verwendung gemäß Anspruch 9, worin es sich bei der Erkrankung, der Störung oder dem Zustand um Parkinson handelt.

11. Zusammensetzung zur Verwendung gemäß Anspruch 9 oder 10, worin die Zusammensetzung im Wesentlichen kontinuierlich verabreicht wird.

12. Zusammensetzung zur Verwendung gemäß einem beliebigen der Ansprüche 9 bis 11, worin die Zusammensetzung subkutan verabreicht wird.

13. Zusammensetzung zur Verwendung gemäß einem beliebigen der Ansprüche 9 bis 12, worin die Zusammensetzung einem Menschen verabreicht wird.

14. Zusammensetzung zur Verwendung gemäß einem beliebigen der Ansprüche 9 bis 13, worin die Zusammensetzung mit einer Geschwindigkeit von 0,01 ml/Stunde/Stelle bis 0,4 ml/Stunde/Stelle verabreicht wird.

15. Zusammensetzung zur Verwendung gemäß Anspruch 14, worin die Zusammensetzung mit einer Geschwindigkeit von 0,16 ml/Stunde/Stelle bis 0,24 ml/Stunde/Stelle verabreicht wird.

16. Zusammensetzung zur Verwendung gemäß einem beliebigen der Ansprüche 9 bis 11, worin die Zusammensetzung intraduodenal mit einer Geschwindigkeit von 1,0 ml/Stunde während des Tages und mit einer Geschwindigkeit von 0 ml/Stunde bis 0,5 ml/Stunde während der Nacht verabreicht wird.

## Revendications

1. Composition liquide pharmaceutiquement acceptable comprenant :
environ 0,75 % en poids de carbidopa ;
environ 6 % en poids de lévodopa ;
d'environ 12 % à environ 36 % en poids d'arginine ;
environ 0,5 % en poids d'acide ascorbique ou d'un sel de celui-ci ;
d'environ 0,1 % à environ 0,6 % en poids de L-cystéine, ou d'un sel de celle-ci, ou d'environ 0,01 % à environ 1 % en poids de N-acétylcystéine (NAC) ; et
facultativement, d'environ 0,1 % à environ 0,5 % en poids, de préférence environ 0,3 % en poids, de polysorbate 80,
dans laquelle ladite composition, après 1 à 24 heures à 25 °C, comprend moins de 0,1 µg/ml d'hydrazine, tel que déterminé par un procédé de CGSM.

2. Composition selon la revendication 1, dans laquelle la composition a moins de 1 % en poids d'acide 3,4-dihydroxyphényl-2-méthylpropionique (dégradant), par rapport à la quantité de carbidopa, tel que déterminé par CLHP.

3. Composition selon la revendication 1, dans laquelle ledit sel d'acide ascorbique est l'ascorbate de sodium, l'ascorbate de potassium ou l'ascorbate de calcium.

4. Composition selon la revendication 1, comprenant environ 15,2 % en poids d'arginine, environ 0,5 % en poids d'acide ascorbique, environ 0,4 % en poids de L-cystéine et environ 0,3 % en poids de polysorbate 80.

5. Composition selon la revendication 1, comprenant environ 15,2 % en poids d'arginine, environ 0,5 % en poids d'acide ascorbique, environ 0,5 % en poids de NAC, et environ 0,3 % en poids de polysorbate 80.

6. Composition selon la revendication 1, comprenant 6 % en poids de lévodopa, 0,75 % en poids de carbidopa, 15,2 % en poids d'arginine, 0,5 % en poids d'acide ascorbique, et 0,5 % en poids de NAC.

7. Composition liquide pharmaceutiquement acceptable comprenant :
d'environ 0,6 à 1,4 % en poids de carbidopa ;
environ 6 % en poids de lévodopa ;
d'environ 15 % à environ 16 % en poids d'arginine ;
environ 0,5 % en poids d'acide ascorbique ou d'un sel de celui-ci ;
environ 0,4 % en poids de L-cystéine ou environ 0,5 % en poids de N-acétylcystéine (NAC) ; et
environ 0,3 % en poids de polysorbate 80,
dans laquelle ladite composition, après 1 à 24 heures à 25 °C, comprend moins de 0,1 µg/ml d'hydrazine, tel que déterminé par un procédé de CGSM.

8. Composition selon l'une quelconque des revendications 1 à 7, comprenant moins de 0,1 µg/ml d'hydrazine après 1 à 30 jours, à une température de 25 °C, telle que déterminée par CGSM.

9. Composition liquide pharmaceutiquement acceptable selon l'une quelconque des revendications 1 à 8, pour une utilisation dans le traitement d'une maladie, d'un trouble ou d'une affection associé(e) à la perte de dopamine ou de neurones dopaminergiques.

10. Composition pour une utilisation selon la revendication 9, dans laquelle ladite maladie, ledit trouble ou ladite affection est la maladie de Parkinson.

11. Composition pour une utilisation selon la revendication 9 ou 10, dans laquelle la composition est administrée de manière sensiblement continue.

12. Composition pour une utilisation selon l'une quelconque des revendications 9 à 11, dans laquelle la composition est administrée par voie sous-cutanée.

13. Composition pour une utilisation selon l'une quelconque des revendications 9 à 12, dans laquelle la composition est administrée à un être humain.

14. Composition pour une utilisation selon l'une quelconque des revendications 9 à 13, dans laquelle la composition est administrée à une vitesse de 0,01 ml/heure/site à 0,4 ml/heure/site.

15. Composition pour une utilisation selon la revendication 14, dans laquelle la composition est administrée à une vitesse de 0,16 ml/heure/site à 0,24 ml/heure/site.

16. Composition pour une utilisation selon l'une quelconque des revendications 9 à 11, dans laquelle la composition est administrée par voie intraduodénale à une vitesse de 1,0 ml/heure durant le jour et à une vitesse de 0 ml/heure à 0,5 ml/heure la nuit.
